# EUROPEAN PATENT SPECIFICATION

(11) **EP 4 072 530 B1**
(45) Date of publication and mention of the grant of the patent: **30.08.2023**
(21) Application number: 20820941.1
(22) Date of filing: 10.12.2020
(51) Int. Cl.: A61K 9/127, A61K 31/704, A61K 41/00, A61K 49/00, A61P 35/00, C07D 401/06, A61K 33/24, A61K 31/44, A61K 31/40, A61K 31/09, A61K 9/00, A61K 38/07, A61K 49/18, A61K 47/69

(54) **STIMULI - OR BIO- RESPONSIVE COPOLYMERS, THE POLYMERSOMES COMPRISING THE SAME AND THEIR USE IN DRUG DELIVERY**
STIMULI- ODER BIOREAKTIVE COPOLYMERE, POLYMERSOME DAMIT UND VERWENDUNG DAVON BEI DER ARZNEIMITTELABGABE
COPOLYMÈRES SENSIBLES AUX STIMULI OU BIOSENSIBLES, LES POLYMERSOMES LES COMPRENANT ET LEUR UTILISATION DANS L'ADMINISTRATION DE MÉDICAMENTS

(30) Priority: 10.12.2019 EP 19306612
(43) Date of publication of application: 19.10.2022
(73) Proprietor: Centre national de la recherche scientifique, 75016 Paris (FR); Institut Polytechnique de Bordeaux, 33400 Talence (FR); ECOLE NATIONALE SUPERIEURE DE CHIMIE DE PARIS, 75231 Paris Cedex 5 (FR); INSTITUT NATIONAL DE LA SANTE ET DE LA RECHERCHE MEDICALE - INSERM, 75013 Paris (FR); Université de Bordeaux, 33000 Bordeaux (FR); Université Paris Cité, 75006 Paris (FR)
(72) Inventor: DALKO, Peter I., 91400 ORSAY (FR); SANDRE, Olivier, 33600 Pessac (FR); RAMNICEANU, Grégory, 91400 Tel Aviv (IL); LECOMMANDOUX., Sébastien, 33610 Canejan (FR); CHINOY, Zoeisha, 33800 Pessac (FR); DOAN, Bich-Thuy, 78420 Carrieres sur Seine (FR); DUNKEL, Petra, 1094 Budapest (HU); KUMAR, Amit, 75006 Paris (FR)
(74) Representative: Lavoix
(86) International application number: PCT/EP2020/085615
(87) International publication number: WO 2021/116331

(56) References cited:
- WO-A1-2010/049611
- WO-A1-2017/173453
- XIN MU ET AL: "Stimulus-responsive vesicular polymer nano-integrators for drug and gene delivery", INTERNATIONAL JOURNAL OF NANOMEDICINE, vol. Volume 14, 1 July 2019 (2019-07-01), pages 5415-5434, XP055693811, AUCKLAND, NZ ISSN: 1176-9114, DOI: 10.2147/IJN.S203555 cited in the application

## Description

The present invention concerns the field of polymersomes, and their uses in therapeutics.

Polymersomes are artificial vesicles that may vehicle an aqueous solution in the core, surrounded by a bi-layer membrane. These carriers are prepared by the self-assembly of amphiphilic block copolymers containing two or more chemically distinct (monomer) sequences joined by a covalent bond that prevents phase separation of the blocks upon dissolution. Due to their hollow and spherical morphology, polymersomes are capable of encapsulating various agents within the vesicle core or in the hydrophobic bilayer depending on the characteristics of the payload. The aqueous core of polymersomes is separated from the outside medium by a hydrophobic membrane, which makes them a vesicle-like structure with an aqueous inner core surrounded by a hydrophobic shell. Thus, this makes them suitable as drug carriers for hydrophilic substances (in the lumen) and hydrophobic substances (in the membrane) possibly offering a multipurpose cocktailtreatment or a diagnostic-therapy combination for biomedical applications. In particular, scientific literature contains several descriptions of the insertion of magnetic iron oxide nanoparticles coated with a hydrophobic surfactant shell within the membranes of polymersomes (Lecommandoux et al, Adv. Mat 17 (6), 712-718 (2005)). Polymersomes that are degradable by pH or by enzymatic route (esterases) have been reported earlier (Mu et al, International Journal of Nanomedicine 2019:14 5415-5434).

According to the most common delivery strategies, hydrophobic blocks can be progressively degraded, or, converted into hydrophilic substances, or, cleavable linkages are incorporated between hydrophobic and hydrophilic blocks. As the degradation and thus the delivery are based on similar concepts than those used in other prodrug strategies, the passive release i.e. utilization of the target tissue physiology for the delivery of the active compounds suffers similar shortcomings, and is often very slow.

It is therefore desirable to provide alternative strategies for active delivery.

The invention provides stimuli- or bio- responsive polymersomes including a photo- or redox-sensitized composite that may release their content only at the site of action, upon an external stimulus, compatible with therapeutic considerations, or an endogenous stimulus (e.g. peculiar redox potential of tumor microenvironment). These nano-materials undergo chemical bond fragmentation by local electron-transfer (ET) reaction, generated by e.g. the interaction of X-ray/gamma rays, water, and photosensitizing nanoparticles embedded within the polymeric composite matrix.

The present invention thus proposes a novel type of delivery method that may trigger remotely these nanoparticles in high spatial (local) selectivity, resulting in diminished (general) toxicity of the payload. It thus provides a drug delivery system that allows space and time-controlled release of an (active) compound in otherwise inaccessible (body) spaces.

According to an object, the present invention provides a photo- or redox-cleavable amphiphilic copolymer compound of formula (I):

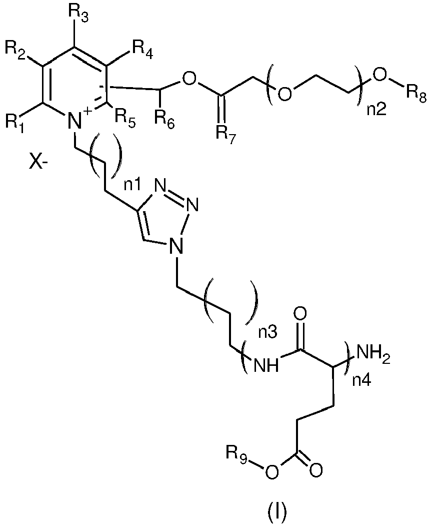

Where:
The side chain 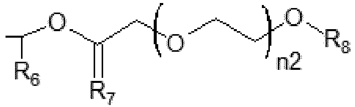 is located at any position of the R₁, R₂, R₃ R₄ and R₅, wherein said R₁, R₂, R₃ R₄ and R₅ is then absent;
When present, R₁, R₂, R₃ R₄ and R₅ are substitution groups on the pyridinium ring and are chosen from H, OH, OMethyl, CN, NR₂, NO₂, halogen, or two adjacent R₁, R₂, R₃ R₄ and R₅ are linked together to form one or more aromatic rings fused with the pyridinium ring to which they are attached so as to form an optionally bi-cyclic substituted quinolinium or an optionally substituted fused tri-cyclic N-containing heteroaryl (e.g. acridinium),
R₆ is H or an alkyl group;
R₇ is O or alkylene;
R₈ and R₉ identical or different are independently chosen from H, alkyl, aryl or benzyl, preferably methyl or benzyl;
n₁ and n₃ are identical or different integers comprised between 0 and 5 defining the length of the spacer;
n₂ is an integer comprised between 34 and 80 denoting the polymerization degree of ethylene oxide in the hydrophilic block of the amphiphilic copolymer;
n₄ is an integer chosen between 18 and 40 denoting the polymerization degree of benzyl-L-glutamate in the hydrophobic block of the amphiphilic copolymer;
X⁻ is a halide or a trifluoromethanesulfonate (⁻OTf), phosphate, sulfate, perchlorate or nitrate ion;
or an alternative pharmaceutically acceptable salt thereof.

The term "photo- or redox-cleavable" means that the copolymer may be cleaved i.e. dissociated into fragments by a stimulus chosen from UV light, visible or near infrared photon (photodynamic), ionizing (beta ray, X-ray or gamma) irradiation, ultrasound or an endogenous or exogenous redox activation.
Typically, the copolymer may be fragmented so as to release each of the side chains substituted on the N-containing heterocyclic core.

As used herein, "amphiphilic" designates the capacity of the copolymer to exhibit both hydrophilic and lipophilic (or hydrophobic) properties and subsequently the ability to self-assemble into multi-molecular objects dispersed in pure water or in aqueous solutions.

"Halo", "hal" or "halogen" refers to fluorine, chlorine, bromine or iodine atom.

"Halide" refers to the anion of a halogen atom.

"Alkyl" represents an aliphatic-hydrocarbon group which may be straight or branched having 1 to 6 carbon atoms in the chain. In a particularly preferred embodiment, the alkyl group has 1 to 4 carbon atoms in the chain. Exemplary alkyl groups include methyl, ethyl, n-propyl, iso-propyl, iso-butyl, n-butyl, tert-butyl, n-pentyl, 3-pentyl.

Alkylene refers to an alkyl group where two hydrogen atoms have been removed at the same carbon atom, so as to have two valencies in the form of a terminal double bond, available for attachment. As illustrated for R7, it may represent a CH₂ or CH₂-CH₃, attached to C through a double bond.

"Aryl" designates an aromatic, mono or bicyclic group comprising between 5 and 10 carbon atoms, such as phenyl or naphthyl.

"Heteroaryl" corresponds to an aromatic, mono, bi or tricyclic group comprising between 3 and 15 carbon atoms and between 1 and 10 heteroatoms, such as nitrogen, oxygen or sulfur atoms. Examples of such heteroaryl groups include pyrrolyl, pyridyl, pyrazolyl, thienyl, pyrimidinyl, pyrazinyl, tetrazolyl, indolyl, quinolinyl, purinyl, imidazolyl, thienyl, thiazolyl, benzothiazolyl, furanyl, benzofuranyl, 1,2,4-thiadiazolyl, isothiazolyl, triazoyl, tetrazolyl, isoquinolyl, benzothienyl, isobenzofuryl, pyrazolyl, carbazolyl, benzimidazolyl, isoxazolyl, acridinyl, etc.

"Benzyl" abbreviated as "Bn" designates an -Alkyl-phenyl group, where Alkyl is defined as above.

In the formula (I), the polyethylene oxide) block exhibits hydrophilic properties, while the 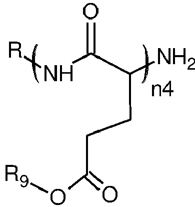 poly(glutamate) moiety exhibits lipophilic properties in particular if R₉ is different from H (i.e. the side chains forms an ester bond, although poly(glutamic acid) can also form hydrophobic alpha-helices structures when the carboxylic group is protonated, i.e. at acidic pH below its pKa).

Preferably, in general formula (I) :
R₅ is absent and the side chain 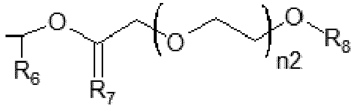 is located on the R₅ (ortho) position; and/or
R₁, R₂, R₃ and R₄ represent H, or R₁, R₂ are linked together to form a phenyl ring fused with the pyridinium ring to which they are attached so as to form an optionally substituted quinolinium and R₃ and R₄ are H; and/or
R₆ is H or methyl;
R₇ is O, or -(CH=R₇)- represents -(CH=CH₂)_{-;} and/or
R₈ and R₉ identical or different are independently chosen from alkyl or benzyl, more preferably R₈ is alkyl, such as methyl, and R₉ is benzyl; and/or
n₁ and n₃ are identical or different integers comprised between 0 to 5; and preferably equal to 1 or 2, defining the length of the spacer; and/or
n₂ is an integer comprised between 34 and 80 denoting the polymerization degree of ethylene oxide in the hydrophilic block of the amphiphilic copolymer; and/or
n₄ is an integer chosen between 18 and 40, more preferably 20-35; and/or
X⁻ is a halide or a trifluoromethanesulfonate (OTf⁻) ion.

According to an embodiment, the copolymer compound of formula (I) may be chosen from the compounds of formulae: 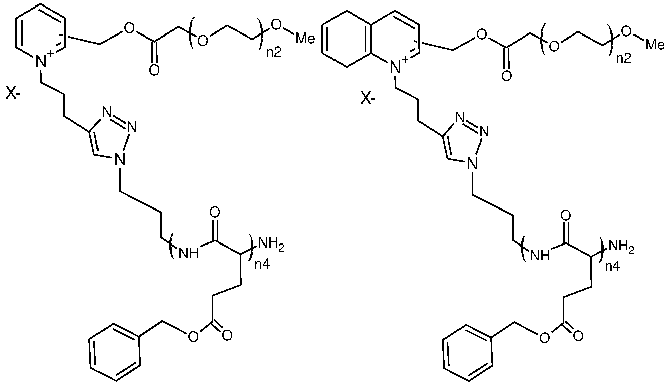 ; more preferably from the following compounds: 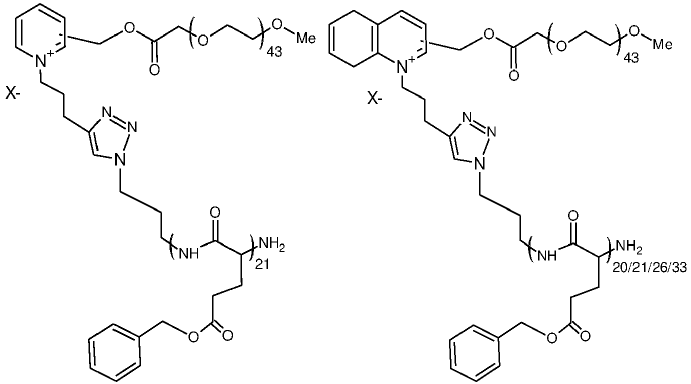 Where X⁻ is a halide or a trifluoromethanesulfonate (OTf),
And the alternative pharmaceutically acceptable salts thereof.

The compounds of Formula (I) comprise amphiphilic diblocks, such as poly(benzyl-L-glutamate) (PBLG) as the hydrophobic block (prepared from the corresponding L-amino acid N-carboxyanhydride (NCA) by ring-opening polymerization (ROP), and such as polyethylene glycol) (PEG) also called polyethylene oxide) (PEO) as the hydrophilic block. These blocks are assembled by chemical bond coupling around a redox-sensitive linker derived from N-alkyl heterocycles, such as picolinium, or, quinolinium derivatives.

The present invention includes salts of compounds of formula (I). The salts may preferably be pharmaceutically acceptable salts. Lists of suitable salts may be found in Remington's Pharmaceutical Sciences, 17th ed., Mack Publishing Company, Easton, PA, 1985, p. 1418 and P.H. Stahl, C.G. Wermuth, Handbook of Pharmaceutical salts - Properties, Selection and Use, Wiley-VCH, 2002.

These salts are advantageously prepared with pharmaceutically acceptable acids, but salts with other acids, useful for example for the purification or for the isolation of the compounds of formula (I), also form part of the invention.

The compounds of formula (I) can comprise one or more asymmetric carbon, in particular the α carbon of the amino acid benzyl-glutamate that is preferably in its natural L configuration, hence the PBLG denomination for the block. However they can exist in the other forms of enantiomers or diastereoisomers. These enantiomers and diastereoisomers, as well as their mixtures, including racemic mixtures, form part of the invention. It is well known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

According to a further object, the present invention also concerns the process of preparation of the copolymer of formula (I), said process comprising the step of reacting a compound of formula (II): 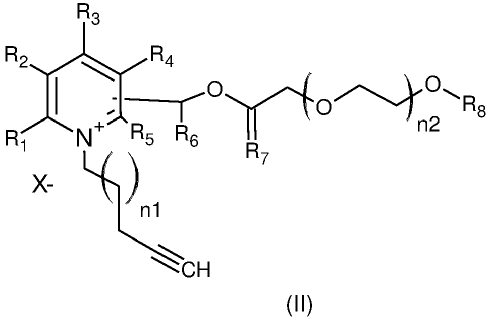 with a compound of formula (III): 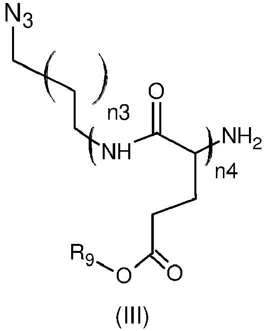 Wherein R₁, R₂, R₃ R₄, R₅, R₆, R₇, R₈, R₉, R, X, n₁, n₂ n₃ and n₄ are defined as in formula (I).

The compounds of the present invention and intermediates thereof may be prepared in a number of ways well known to those skilled in the art. The compounds can be synthesized, for example, by application or adaptation of the methods described below, or variations thereon as appreciated by the skilled artisan. The appropriate modifications and substitutions will be readily apparent and well known or readily obtainable from the scientific literature to those skilled in the art. In particular, such methods can be found in R.C. Larock, Comprehensive Organic Transformations, VCH publishers, 1989*.* The reagents and starting materials may be commercially available, or readily synthesized by well-known techniques by one of ordinary skill in the arts. All substituents, unless otherwise indicated, are as previously defined.

In the reactions described hereinafter, it may be necessary to protect reactive functional groups, for example hydroxy, amino, imino, thio or carboxy groups, where these are desired in the final product, to avoid their unwanted participation in the reactions. Conventional protecting groups herein named Pg may be used in accordance with standard practice, for examples see T.W. Greene and P. G. M. Wuts in Protective Groups in Organic Synthesis, John Wiley and Sons, 1991; J. F. W. McOmie in Protective Groups in Organic Chemistry, Plenum Press, 1973.

Some reactions may be carried out in the presence of a base. There is no particular restriction on the nature of the base to be used in this reaction, and any base conventionally used in reactions of this type may equally be used here, provided that it has no adverse effect on other parts of the molecule. Examples of suitable bases include: sodium hydroxide, potassium carbonate, triethylamine, alkali metal hydrides, such as sodium hydride and potassium hydride; alkyllithium compounds, such as methyllithium and butyllithium; and alkali metal alkoxides, such as sodium methoxide and sodium ethoxide.

Usually, reactions are carried out in a suitable solvent. A variety of solvents may be used, provided that it has no adverse effect on the reaction or on the reagents involved. Examples of suitable solvents include: hydrocarbons, which may be aromatic, aliphatic or cycloaliphatic hydrocarbons, such as hexane, cyclohexane, benzene, toluene and xylene; amides, such as dimethyl-formamide; alcohols such as ethanol and methanol and ethers, such as diethyl ether and tetrahydrofuran.

The reactions can take place over a wide range of temperatures. In general, it was found convenient to carry out the reaction at a temperature of from 0°C to 150°C (more preferably from about room temperature to 100°C). The time required for the reaction may also vary widely, depending on many factors, notably the reaction temperature and the nature of the reagents. However, provided that the reaction is effected under the preferred conditions outlined above, a period of from 3 hours to 20 hours will usually suffice.

The compound thus prepared may be recovered from the reaction mixture by conventional means. For example, the compounds may be recovered by distilling off the solvent from the reaction mixture or, if necessary after distilling off the solvent from the reaction mixture, pouring the residue into water followed by extraction with a waterimmiscible organic solvent and distilling off the solvent from the extract. Additionally, the product can, if desired, be further purified by various well-known techniques, such as recrystallization, precipitation, or by various chromatography techniques, notably column chromatography or preparative thin layer chromatography.

Generally, the starting products and the reagents described herein are commercially available or can be prepared according to the examples, or by application or adaptation of known methods.

According to a further object, the invention also concerns an aqueous suspension of polymersomes comprising an amphiphilic bilayer, wherein the amphiphilic bilayer of said polymersomes comprise a photo-cleavable or redox-cleavable amphiphilic copolymer compound and embedded nanoparticles acting both as photosensitizers and contrast agents for image-guided therapy according to the invention.

Said polymersome is also called herein "amphiphilic copolymer vesicle".

The present invention also concerns the preparation of polymersomes. The compounds of the invention may be able to self-assemble into a polymersome suspension in water (i.e) a large number such as hundreds of millions of polymersomes per microliter suspended in an aqueous buffer (as typically measured with the qNano counter instrument ilZON Science inc.), by means of the amphiphilic nature of the building blocks of the macromolecules (e.g. having large hydrophilic and hydrophobic terminals) for which water acts a "selective solvent" (i.e. a good solvent of one block only and a bad solvent for the other one). Amphiphilic compounds are known to undergo self-assembly into a wide range of morphologies like spheres, cylinders, continuous structure, lamella, vesicles or many other complex shapes/assemblies. The hydrophilic and hydrophobic blocks of Formula (I) allows self-assembly by forming polymersomes, in particular thanks to the antagonist miscibility in aqueous environment of both blocks. Among different self-assembly processes, nanoprecipitation also called 'solvent shift' or 'solvent displacement' method consists in adding a selective solvent, usually water, that is a good solvent for one block and a bad solvent for the other block, to a copolymer solution initially dissolved in a nonselective solvent or common solvent (THF, DMSO, acetone, alcohol...) that is a good solvent of both blocks and that, in addition, is itself miscible with water. After the process, the excess of solvent mixed with water is eliminated either by slight heating (e.g. 40°C overnight) or by dialysis in a tube with a semi-permeable membrane that is equilibrated with an aqueous solution (pure water, pH buffer, saline, cell culture medium, blood plasma, etc.).

Sizes and morphologies of polymersomes can be controlled by varying the chemical constitution and block length of the co-polymer, e.g. by the chemical step, but also by the preparation method or self-assembly process, e.g. by controlling the properties of the initial co-polymer solution such as concentration, temperature, and solvent type, pH and salinity of the aqueous phase, etc., and by the mixing conditions (stirring velocity, fast addition or slow addition, at controlled flowrate, water-in-solvent addition or reverse order, macroscopic container or microfluidic reactor...) as referenced for instance in C. Sanson, C. Schatz, J-F. Le Meins, A. Brûlet, A. Soum, S. Lecommandoux, Langmuir 2010, 26, 2751-2760.

According to the invention, polymersomes may be prepared in the size range of tens of nanometers to less than 10 micrometers of diameter. Typically, the average diameter is comprised between 50 nm and 1 micrometer, as determined by the so-called 2^{nd} order Cumulant fitting method of the dynamic light scattering (DLS) curves.

The envelope bilayer is generally 10 to 30 nm thick, as measured by electron microscopy or by a small angle scattering technique (X-ray or neutrons, respectively SAXS or SANS).

Typically, the polymersome suspension of the invention exhibits narrow size distribution width, of about 80-140 nm of hydrodynamic radii with polydispersity index (PDI) between 0.10 and 0.25 as measured by DLS, and/or correlated to other types of hydrodynamic size analysis such as Coulter counter or nanoparticle tracking (NTA). The nanoscale structure of the polymersomes may be ascertained by transmission (TEM) or scanning (SEM) and/or cryogenic (cryo-TEM) electron microscopy image analyses, with or without positive or negative sample staining, or by atomic force microscopy (AFM). They may be characterized also by small angle X-ray scattering (SAXS), small angle neutron scattering (SANS), asymmetric flow (A4F) or field-flow fractionation (FFF) chromatography or multi-angle static and dynamic light scattering (MALS) techniques to determine the radius of gyration (*R*_{g}) in addition to the hydrodynamic radius (*R*ₕ) of the self-assemblies. Then the so-called p-ratio of *R*_{g} over *R*ₕ enables discriminating vesicular (shell-like) structure by a value close to 1, as opposed to values close to √(3/5)~0.77 for filled spherical micelles or reaching up to 2 for elongated (rod-like) cylindrical micelles. These procedures may be conducted by application or adaptation of known methods in colloidal science.

According to an embodiment, said polymersome structure further comprises a significant number of (semi-)metal or metal oxide or other metal chalcogenide (sulfide, telluride...) nanoparticles embedded into said polymersome membrane, constituting a significant feed weight ratio (FWR) relatively to the total polymer weight, typically from 5% to 50%, and preferentially between 10% and 20%.

Typically, said mineral i.e. non-organic metal, semi-metal, metal oxide or chalcogenide, alkaline earth or rare earth may be chosen from the d group consisting of a transition metal or an element of another family yet with an atomic number greater than 21, a noble metal (silver, gold, platinum...), a metal alloy, or a lanthanide of the f-group of the periodic table of the elements.

According to a further embodiment, said mineral (metal, semi-metal, metal oxide, chalcogenide, or an earth) nanoparticle is an ultra-small iron oxide nanoparticle (USPIO), a superparamagnetic iron oxide nanoparticle (SPION), a very small iron oxide nanoparticles (VSION), a hafnium-oxide, an iron-bismuth or iron-platinum alloy, a gadolinium oxide, silver, platinum, or a gold nanoparticle or atom cluster.

Superparamagnetic iron oxide nanoparticles (SPION) are typically used as MRI contrast agents and are composed of nano-sized iron oxide crystals coated with dextran or carboxydextran. The principal effect of USPIO or SPION particles is on T₂* relaxation and thus magnetic resonance (MR) imaging is usually performed using T₂/T₂*-weighted sequences in which the tissue signal loss is due to the susceptibility effects of the superparamagnetic iron oxide core. Beyond their MR imaging capacity, USPIO and SPION nanoparticles are studied in cancer therapy. USPIOs showed interesting radiation dose deposit profile in the past, some of them by intracellular manner. Iron oxide nanoparticles (USPIOs or SPIONs) for example allow bio-distribution study by *in vivo* MRI and EPR (enhanced retention effect) monitored by MRI. In addition to regular SPIONs usually used as negative (T₂/T₂*-type) MRI contrast agents, there exists also very small iron oxide nanoparticles (VSION), with diameters around 4±1 nm, that can be used as positive contrast agents (signal enhancer) in combination with T₁-weight sequences, in particular for MRI angiography [T Vangijzegem, D Stanicki, S Boutry, Q Paternoster, L Vander Elst, R N Muller, S Laurent, Nanotechnology 29, 2018, 265103]

USPIO, SPION and VSION nanoparticles may act both as MRI contrast agents, and as X-ray or Γ-ray photosensitizers. This may allow for *in vivo* bio-distribution studies such as for mapping tumors by MRI, before applying conventional radiotherapy. These polymersomes may be used for encapsulation of reporters, such as near infrared (NIR) fluorescent dyes, or, active pharmaceutical ingredients (API). They thus may be useful for monitoring the bio-distribution and studying the activation of stimuli-sensitive polymersomes.

According to a still further embodiment, said polymersomes may further comprise a API, said API being encapsulated within either the aqueous compartment or within the hydrophobic membrane of said polymersomes, depending on water solubility of said API.

The API encapsulated by the polymersomes may be chosen from APIs for the treatment of cancer, immune-related diseases, inflammation, diabetes, bacterial and/or viral infections, and orphan diseases, pediatric or age-related diseases, such as Sorafenib^{®} Doxorubicin, Monomethyl auristatin E (MMAE), cisplatin, or Combretastatine^{®}.

The invention also concerns the process of preparation of the polymersomes of the invention.

According to the invention, the polymersomes, optionally comprising the API and/or the mineral (metal or metal oxide) nanoparticles may be obtained by self-assembly. Typically, this may be achieved by solvent displacement, by application or adaptation of the methodology disclosed by Sanson et al ACS Nano, 2011, 5, 1122-1140. According to an embodiment, this may be conducted in mixture of water and a miscible organic solvent such as tetrahydrofuran (THF), dimethylsulfoxide (DMSO), acetone, isopropylalcohol (iPrOH), ethanol (EtOH), acetonitrile (ACN), dimethylformamide (DMF) or ethylacetate (EtOAc). Typically, the mixture comprises between 10 and 70 % v/v of the organic solvent and between 30 and 90 % of water.

Polymersomes whose membranes comprise a significant weight fraction of mineral (metal or metal oxide) nanoparticles may be prepared by water displacement methodology, advantageously, by using lipophilic surfactants (such as phosphoric ester of polyethylene oxide)₉ nonylphenyl ether) sold under brand name Beycostat^{™} typically for the metal oxide nanoparticle such as ultra-small iron oxide particle (USPIO) coating or poly(2-vinyl pyridine) (P2VP) for silver or gold nanoparticle coating and dispersion in THF.

According to a further object, the present invention concerns an aqueous suspension comprising polymersomes according to the invention.

According to a still further object, the invention also provides for the process of preparation of the suspension of polymersomes of the invention, said process comprising the steps of:
- Optionally mixing a compound of formula (I) with a hydrophobically coated metal or metal oxide nanoparticle suspension and/or a API in an organic solvent miscible with water
- Self-assembly by nanoprecipitation of the polymersome vesicles in an aqueous solution (also called "solvent-shift" method), optionally followed by
- dialysis or heating of the suspension to remove any trace of organic solvent.

Said water-miscible solvent may be typically chosen from THF, DMSO, DMF, acetonitrile, acetone, ethyl acetate, or various alcohols.

According to a still further object, the present invention also concerns a pharmaceutical composition or a medical device comprising an aqueous suspension of the invention. According to a further object, the present invention also concerns the use of the photo-cleavable or redox-cleavable amphiphilic copolymer compound, the polymersome, the suspension of polymersomes, the medical device or the pharmaceutical composition of the invention as a contrast agent for biomedical imaging.

Said contrast agent may be particularly suitable for a bio-imaging modality, optical endoscopy, ultrasound echography, magnetic resonance imaging (MRI), X-ray scanner also called computed tomography (CT), particularly when comprising an API for the image-guided treatment of a disorder. Said treatment may typically comprise:
- the implantation of said medical device at a disorder site or the systemic administration of said compound, polymersome, suspension of polymersomes or pharmaceutical composition;
- the activation of the compound of formula (I) at the targeted site by either an externally applied mean or an endogenous signal, thereby releasing the API at the targeted site; and
- optionally monitoring the distribution of the mineral (metal or metal oxide) nanoparticles embedded in polymersomes within the human body.

Typically, the activation may be achieved by the cleavage of the polymersome membrane, said cleavage being induced by UV light, visible photon (photodynamic), ionizing (beta ray, X-ray or gamma-ray) irradiation, focused ultrasound (FUS), or, either endogenous or exogenous redox activation at the targeted site. Therefore, the activation may be carried out by beta rays, X rays or gamma rays irradiation, or by exploiting endogenous signals such as redox enzymes, metalloproteases, reactive oxygen species (ROS) like peroxides or reactive nitrogen species (NO-). The activation i.e. the external (trigger) signal may affect some physicochemical e.g. rheological (i.e. fluid-like) or permeability parameters of the membrane, and the structure or morphology of the polymersomes of the invention. The modification can affect the ratio between the hydrophilic or hydrophobic blocks, the solubility of the membrane components, the degradation or bond cleavage of the macromolecules, which would result in changes of their aggregation features at the supramacromolecular level. The response to the stimuli might be reversible, or, irreversible, depending on the employed approach.

The local activation leads to minimized toxicity to vital tissues and organs away from the target. The combination of intracellular high-energy dose deposit and of the local liberation of chemical API substances opens new protocols in cancer personalized therapy and may change the benefit / risk ratio in radiotherapy.

Typically, the monitoring may be achieved by a non-invasive bio-imaging technique such as MRI with a magnetic metal or metal oxide or lanthanide (Gd, Dy...), optical modalities (visible or near-infrared fiber optic endoscopy or photoluminescence with quantum dots (QD) or lanthanide doped up-converting nanoparticles (UCNP), positron emission tomography (PET), single photon emission computed tomography (SPECT) with a radionuclide, or X-ray computed tomography with an element of atomic number higher than 21 or with a halogen, such as iodine, or fluorine for ¹⁹F MRI with triflate as the counter-anion of the quaternarized heterocycle such as pyridinum, quinolinium ring.

The method of the invention relies on the generation of local electrons (Auger-, or Compton-electrons) formed by the interaction of high energy electromagnetic wave (X-rays / gamma-rays) and metal-derived nanoparticles embedded in the polymeric matrix. Besides, in the case of a magnetic transition metal (e.g. iron) or magnetic lanthanide (e.g. gadolinium, dysprosium,...) the proton relaxivity properties of the resulting MRI contrast agent is high, leading to high efficacy for *in vivo* MRI for image guided study or therapy. Otherwise, high atomic number atoms (e.g. iodine, silver, gold, barium, bismuth...) can provide absorption or phase contrast for imaging by computed (X ray) tomography.

The disorder may be chosen in particular from cancer, immune-related diseases, inflammation, diabetes, bacterial and/or viral infections, pediatric or age-related diseases.

According to a further embodiment, the present invention also concerns a method of treatment and/or prevention of a disorder chosen from cancer, immune-related diseases, inflammation, diabetes, bacterial and/or viral infections, pediatric or age-related diseases comprising the administration of a compound of formula (I) according to the invention as defined above to a patient in the need thereof.

As used herein, the term "patient" refers to a warm-blooded animal such as a mammal, preferably a human or a human child, which is afflicted with, or has the potential to be afflicted with one or more diseases and conditions described herein.

As used herein, a "therapeutically effective amount" refers to an amount of a compound of the present invention, which is effective in reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment and chronic use.

As used herein, the expression "pharmaceutically acceptable" refers to those compounds, materials, compositions, or dosage forms, which are, within the scope of sound medical judgment, suitable for contact with the tissues of human beings and animals without excessive toxicity, irritation, allergic response, or other problem complications commensurate with a reasonable benefit/risk ratio.

In the context of the invention, the term "treating" or "treatment", as used herein, means reversing, alleviating, inhibiting the progress of, or preventing the disorder or condition to which such term applies, or one or more symptoms of such disorder or condition.

The dosage of the API to be administered depends on such variables as the type and extent of progression of the disease or disorder, the overall health status of the particular patient, the relative biological efficacy of the compound selected, and formulation of the compound, excipients, and its route of administration.

Compounds of the present invention may be formulated into a pharmaceutically acceptable preparation, on admixing with a carrier, excipient or a diluent, in particular for oral or parenteral use. Oral preparations may be in the form of tablets, mini-tablets, capsules, topical or parenteral. A solid carrier can include one or more substances which may also act as flavoring agents, lubricants, solubilizers, suspending agents, fillers, glidants, compression aids, binders or tablet-disintegrating agents; it can also be an encapsulating material. Liquid carriers can include water, an organic solvent, a mixture of both or pharmaceutically acceptable oils and fats (e.g. medium-chain triglycerides). The compositions may conveniently be administered in unit dosage form and may be prepared by any of the methods well known in the pharmaceutical art, for example, as described in Remington: The Science and Practice of Pharmacy, 20th ed.; Gennaro, A . R., Ed.; Lippincott Williams & Wilkins: Philadelphia, PA, 2000. Pharmaceutically compatible binding agents and/or adjuvant materials can be included as part of the composition.

The tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Preferred tablets contain lactose, cornstarch, magnesium silicate, croscarmellose sodium, povidone, magnesium stearate, or talc in any combination. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bi-modal.

Liquid preparations for administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, acrylate copolymers, vegetable oils such as olive oil, and organic esters such as ethyl oleate. Aqueous carriers include mixtures of alcohols and water, hydrogels, buffered media, and saline. In particular, biocompatible, biodegradable *L*-lactide polymer (PLA), *L*-lactide-co-glycolide copolymer (PLGA), or polyethylene oxide)-*co*-poly(propylene oxide) copolymers may be useful excipients to control the release of the API . Intravenous vehicles can include fluid and nutrient replenishers, electrolyte replenishers, such as those based on Ringer's dextrose, and the like. Other potentially useful parenteral delivery systems for these active compounds include ethylene-vinyl acetate copolymer particles, osmotic pumps, implantable infusion systems, hydrogels and liposomes.

Other features of the invention will become apparent in the course of the following description of exemplary embodiments that are given for illustration of the invention and not intended to be limiting thereof.

### Description of the Figures:

Figure 1 illustrates the determination of the half maximal inhibitory concentration (IC50) values on MCF-7 human breast cancer (A, C) and Ovar-3 carcinoma cell lines (B, D) for two anticancer APIs, combretastatin (A, B) and doxorubicin (C, D) either encapsulated in Quino-Ps polymersomes or as free API, showing increase of IC50 by 2 to 3 orders of magnitude.
Figure 2 shows the relaxometric measurements, by using 7T MR images of USPIO-embedding polymersomes by increasing Fe concentrations (0 to 1 mM) in saline (0.9% NaCl). (a) T₁ weighted (b) T₂ weighted (c) T₂* weighted MR Images.
Figure 3 represents the MRI dynamic susceptibility contrast (DSC) images recorded in living mice at different time points to follow the kinetic uptake and clearance of the magnetic Pico-Ps(Fe) in liver, spleen, kidney, vessels, muscle; (n=3).
Figure 4 represents the T₂-weighted MR images of mice i. liver and ii spleen recorded a) before and b-d) after injection of Quino-Ps(Fe) (20% FWR) (a) 0 min (b) 40 min, (c) 48 h, (d) 7 days.
Figure 5. Variation of the fluorescence by the treatment of Quino-Ps-DOX with added glutathione (GSH) (10 mM).
Figure 6. Calibration curve for the quantification of the doxorubicin release by GSH.

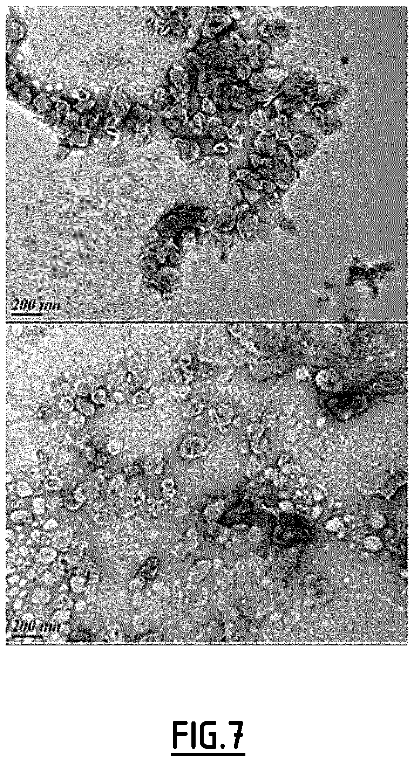

Figure 7. TEM analysis of Quino-Ps samples after irradiation by the ID17 biomedical beamline at European synchrotron radiation facility (ESRF, Grenoble, France) (conditions: Cuvette: quartz, d (light path): 1 mm, Beam energy: 120 keV, Bunch length: 48 ps, Bunch repetition rate 5.68 MHz, Dose: 30 Gy).
Figure 8. Variation in hydrodynamic diameter (Zavg diameter and PDI) of Quino-Ps (A) and Quino-Ps (Fe) (B) as measured by DLS.
Figure 9: *In vitro* fluorescence signal obtained with varying concentrations of PICO-GNP6Ps.
Figure 10: *In vivo* blood fluorescence signal over time after injection of PICO-GNP6Ps in mice.
Figure 11: *In vivo* fluorescence imaging of PICO-GNP6Ps.
Figure 12: The fluorescence spectrum of GNP-modified picolinium NPs (λₑₓ = 400 nm).
Figure 13: The variation of the hydrodynamic size (Z_{avg}) of GNP-modified picolinium NPs by DLS over 70 days.

### Exemples of polymersomes according to the invention (therafter abbreviated Ps)

### 1. Chemical synthesis: preparation of picolinium and quinolinium derived redox probes, their incorporation as linkers between the two blocks of the block-copolymers Representative synthetic pathways are depicted in the schemes 1-4 below for representative compounds of formula (I) of the invention:

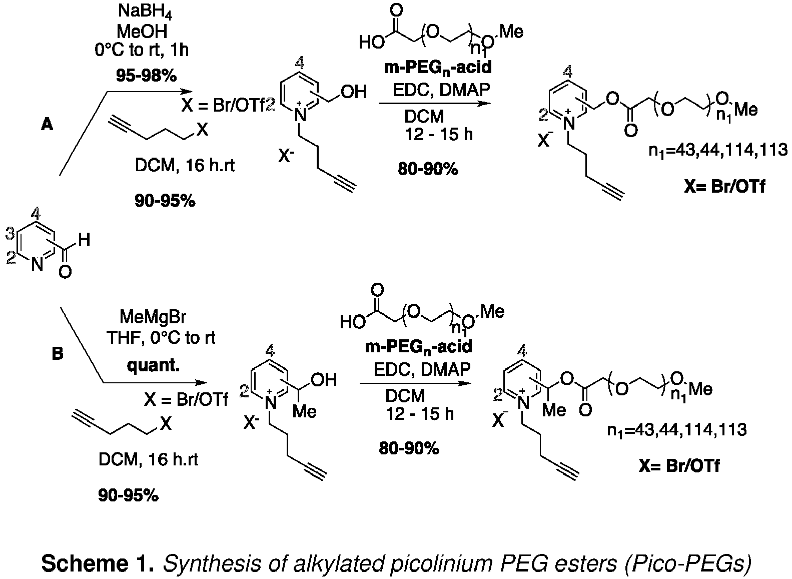

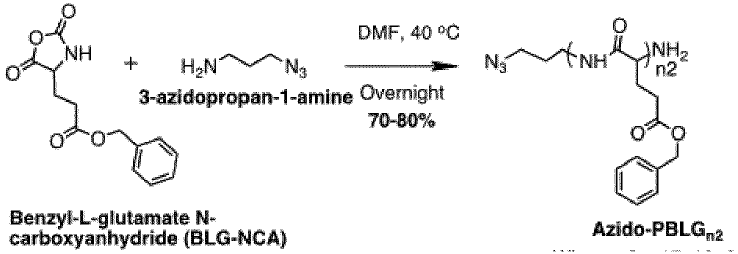

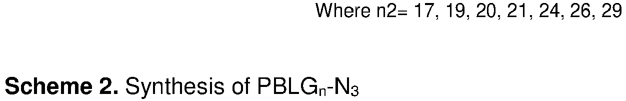

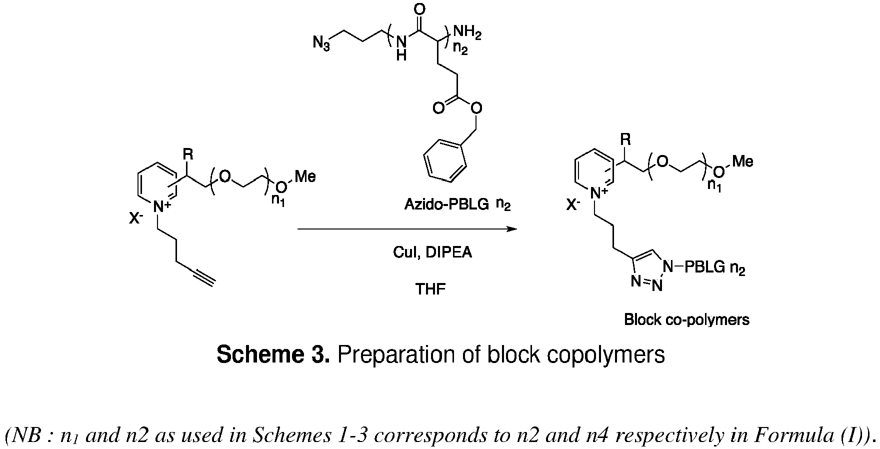

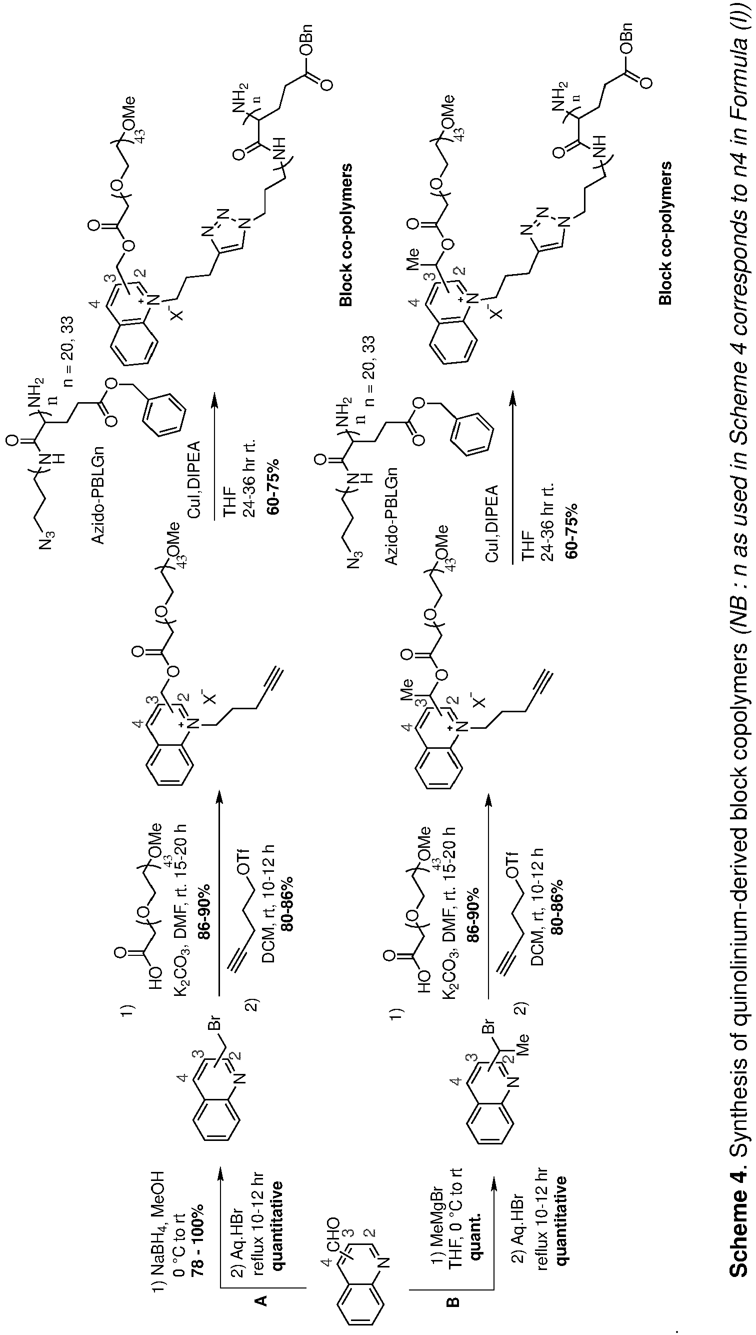

### Generals

Proton nuclear magnetic resonance (¹H NMR) spectra and carbon nuclear magnetic resonance (¹³C NMR) spectra were recorded on a Bruker 250 spectrometer (250 MHz and 63 MHz) and on a Bruker AV-500 spectrometer (500 MHz and 125 MHz). Chemical shifts for protons are reported in parts per million (ppm) downfield from tetramethylsilane (TMS) and are referenced to residual proton in the NMR solvent (CDCl₃: δ 7.26). Chemical shifts for carbon are reported in parts per million downfield from tetramethylsilane and are referenced to the carbon resonances of the solvent CDCl₃ (δ 77.16). NMR spectra recorded in other solvent are indicated in the experimental part.

Data are represented as follows: chemical shift, integration, multiplicity, coupling constants in Hertz (Hz). The following abbreviations are used to set multiplicities: s = singlet, bs = broad singlet, d = doublet, dd = double doublet, ddd = double double doublet, t = triplet, m = multiplet, br m = broad multiplet, bb = broad band.

All solvents and inorganic reagents were from commercial sources and used without purification unless otherwise noted. Thin layer chromatography (TLC) was performed on aluminium-backed Merck Kieselgel 60 F254 precoated plates. The high-performance liquid chromatography (HPLC) analyses were carried out on Waters device 600 with a normal inverse phase column XTerra^{®}_MS C18 (length: 75 mm, diameter: 4.6 mm, stationary phase: 2.5 µm) using a Waters 2487

Dual Absorbance Detector (254-365 nm) and an isocratic / linear gradient system of elution (Methanol-ACN-H₂O 7-2-1 / H₂O-AcONH₄ 10 mM pH 4.6). The volume of injection was 10 µL. The mass analyser was an Agilent from ThermoFisher. The capillary tension was 3.5 kV. The cone tension was 24 V. The temperature of the source was 130 °C and the temperature of desolvatation was 350 °C. Data were treated on ThermoQuest.

### Synthesis of N-methyl picolinium esters

### General procedure for the synthesis of picoline methyl-alcohols.

To a solution of the corresponding pyridine carboxaldehydes (3 mmol) in methanol at 0 °C was added sodium borohydride (1.2 eq.). The mixture was stirred at room temperature (RT) for 1 h before being quenched with HCl (1 M). The solution was then filtered on celite, washed with DCM and concentrated under reduced pressure. The crude product was purified by column chromatography on silica gel (DCM/MeOH 9 / 1) to obtain the corresponding alcohol.

### Pyridin-2-ylmethanol

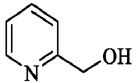

Yellow oil (320 mg, 98%).

**Chemical formula:** C₆H₇NO **Molecular Weight:** 109.12 g·mol⁻¹

**¹H NMR** (CDCl₃, 250 MHz): δ 8.88-8.54 (1H, m), 7.69 (1H, m), 7.29-7.18 (2H, m), 4.75 (2 H, s), 3.16 (1H, bs,).

**¹³C NMR** (CDCl₃, 250 MHz): δ 159.6, 148.4, 136.9, 122.3, 120.8, 64.3.

**MS (ESI):** m/z = 110.0 [M+H]⁺.

### Preparation of 5-bromopent-1-yne

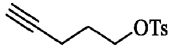

**Pent-4-yn-1-yl 4-methylbenzenesulfonate 29:** In a solution of pent-4-yn-1-ol (1 eq.) in DCM (10 v), was added triethylamine (2.0 eq) and the solution stirred at 0-5 °C for 10 minute, followed by the addition of tosyl chloride (1.2 eq.) at this temperature. The reaction was left to stir for 10 to 15 h at room temperature. The progress was monitored by TLC in EtOAc / cyclohexane (20:80). The reaction was quenched by the addition of water (20 mL) and the product extracted with DCM 2 to 3 times, dried over Na₂SO₄, filtered and concentrated *in vacuo.* Solid mass obtained (off white; 95%).

**¹H NMR** (CDCl₃, 250 MHz): δ 7.82 (d, 2H), 7.38 (d, 2H), 4.18 (t, 2H), 2.46 (s, 3H), 4.77-4.74 (t, 2H), 2.47 (s,1H), 2.30-2.24 (dd, 2H),1.91-1.89 (t, 1H), 1.88-1.82(q, 2H).

### 5-Bromopent-1 -yne

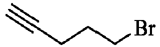

To a solution of pent-4-yn-1-yl 4-methylbenzenesulfonate (1.0 eq.) in DMSO (8 v) was added LiBr (5.0 eq) (exothermicity observed). The reaction was stirred overnight at room temperature. The reaction was quenched by the addition of water (100 mL), followed by stirring for 10 minutes. The product was extracted using DCM 2 to 3 times, dried over Na₂SO₄, filtered and concentrated *in vacuo.* Oily mass obtained (colorless; 72% yield).

**¹H NMR** (CDCl₃, 250 MHz): 3.59-3.53 (t, 2H), 2.45-2.39 (dd, 2H), 2.13-2.05 (q, 2H), 2.03-2.00 (t,1H).

### Preparation of pent-4-yn-1-yl trifluoromethanesulfonate

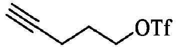

To a solution of pent-4-yn-1-ol (1 eq.) in DCM (10 v), was added pyridine (1.2 eq.) and stirred at 0-5 °C for 5 minutes under inert atm., followed by the addition of triflic anhydride (1.2 eq) at the same temperature. The reaction was stirred for 2 h at room temperature. The reaction was quenched by the addition of water (5 mL) and the product extracted with DCM 2 to 3 times, dried over Na₂SO₄, filtered and concentrated *in vacuo.* Light brown semi solid 81%).

**¹H NMR** (CDCl₃, 250 MHz): 4.62 - 4.56 (t, 2H), 2.78 - 2.72 (dd, 2H), 2.30-2.24 (dd, 2H), 2.18 - 2.12 (m, 3H).

### Quaternarization of various hydroxymethyl picolines

### 2-(Hydroxymethyl)-1-(pent-4-yn-1-yl) pyridin-1-ium

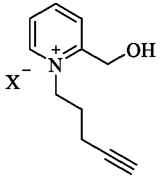

To a solution of **hydroxymethyl** picoline (500 mg, 1.00 eq.) in ACN (3 mL), **pent-4-yn-1-yl triflate** (936 mg, 1.4 equiv.) was added and the solution was stirred at 60 - 70 °C, overnight. Solvent was concentrated *in vacuo* at 40 °C and the crude product was purified by column chromatography using silica gel (DCM: MeOH). Light brown semi solid, 91%.

**Chemical Formula:** C₁₁H₁₄NO⁺ **Molecular Weight:** 176.24 g·mol⁻¹

**¹H NMR** (MeOD, 500 MHz): δ 8.90 (d, *J* = 6.3 *Hz,* 1H), 8.56 (td, *J* = 7.9, 1.5 *Hz,* 1H), 8.25- (d, *J* = 8.4 *Hz,* 1H), 7.98 (ddd, *J* = 7.7, 6.2, 1.6 *Hz,* 1H), 5.03 (s, 2H), 4.68-4.65 (m, 2H), 2.40-2.37 (m,3H), 2.20-2.14 (m, 2H).

### General method for the synthesis of azido poly(γ-benzyl-L-glutamate)

### Synthesis of PBLG₂₁-N₃.

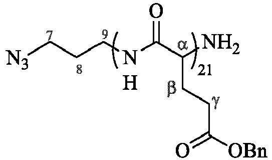

In a glove-box, γ-benzyl-*L*-glutamate *N*-carboxyanhydride (BLG-NCA) (2 g, 7.6 mmol) was dissolved in dry DMF (20 mL). The flask was maintained under argon and brought to the fume-hood, where 1-azido-3-aminopropane (36 µL, 0.362 mmol) was added under argon. The reaction mixture was stirred for 18 h at 40 °C. The polymer was precipitated by slowly adding the reaction mixture into cold Et₂O (200 mL). The precipitate was filtered, washed with cold Et₂O, and dried *in vacuo* to provide PBLG₂₁-N₃ (1.44 g, 86% yield) as a white powder.

**¹H NMR** (90/10 CDCl₃/TFA, 400 MHz,): δ 7.98 - 7.80 (m, 21H, amide-NH), 7.41 -7.22 (m, 105H, aromatic-CH), 5.17 -5.05 - (m, 42H, **Bz**-CH₂), 4.65 - 4.55 (m, 21H, **α**-CH), 3.40 - 3.35 (m, 4H, **7**-CH₂, **9**-CH₂), 2.58 - 2.39 (m, 42H, **γ**-CH₂), 2.22 - 2.04 (m, 21H, **β**-C*H*H), 2.00 - 1.86 (m, 21H, **β**-CH*H*), 1.82 - 1.77 (m, 2H, **8**-CH₂).

**SEC:** Ð = 1.13.

### Synthesis of PBLG-N₃ of different DPs (PBLG₂₀-N₃, PBLG₂₁-N₃, PBLG₂₆-N₃ and PBLG₃₃-N₃)

Ring opening polymerization (ROP) of γ-benzyl-L-glutamate *N-*carboxyanhydride was performed using a 1-azido-3-aminopropane as an initiator to afford α-azido poly(y-benzyl-L-glutamate) (PBLG) (Scheme 5). Initiator 1-azido-3-aminopropane was synthesized as previously reported (Agut et al, Macromol. Rapid Commun. 2008, 29, 1147-1155). PBLG-N₃ polymers of different degrees of polymerization (DPs) were synthesized by ROP in DMF, the reaction being carried for 18h out at 40 °C. For example, for PBLG₂₁-N₃, γ-benzyl-*L*-glutamate *N*-carboxyanhydride (BLG-NCA) (2 g, 7.6 mmol) was dissolved in dry DMF (20 mL) inside a glove-box to protect the NCA from moisture. The flask was maintained under argon and brought to the fume-hood, where 1-azido-3-aminopropane (36 µL, 0.362 mmol) was added under argon. The reaction mixture was stirred for 18 h at 40 °C. The polymer was precipitated by slowly adding the reaction mixture into cold Et₂O (200 mL). The precipitate was filtered, washed with cold Et₂O, and dried *in vacuo* to provide PBLG₂₁-N₃ (1.44 g, 86% yield) as a white powder.

**¹H NMR** (90/10 CDCl₃/TFA, 400 MHz,): δ 7.98 - 7.80 (m, 21H, amide-NH), 7.41 -7.22 (m, 105H, aromatic-CH), 5.17 -5.05 - (m, 42H, **Bz**-CH₂), 4.65 - 4.55 (m, 21H, **α**-CH), 3.40 - 3.35 (m, 4H, **7**-CH₂, **9**-CH₂), 2.58 - 2.39 (m, 42H, **γ**-CH₂), 2.22 - 2.04 (m, 21H, **β**-C*H*H), 2.00 - 1.86 (m, 21H, **β**-CH*H*), 1.82 - 1.77 (m, 2H, **8**-CH₂). **SEC:** Ð = 1.13.

Different DPs were obtained by varying the ratio of monomer to initiator. This provided PBLG₂₀-N₃, PBLG₂₁-N₃, PBLG₂₆-N₃, and PBLG₃₃-N₃ (Table **A**). The DPs were determined by ¹H NMR spectroscopy in 90:10 CDCl₃:trifluoroacetic acid (TFA), based on the integrations of the peaks at -3.35 ppm and -4.59 ppm, where the peak at -3.35 was integrated to 4 as it corresponds to the methylene groups α-to the amide and azide of the initiator, and the peak at -4.59 corresponds to the α-carbon of benzyl glutamate. Size exclusion chromatography (SEC) in DMF was used to obtain the molar mass dispersity (Ð) of 1.05-1.13. SEC chromatograms exhibited a bimodal distribution: Such bimodality for polypeptide chains is usually observed and ascribed to coexistence of helical and random coil structures in solution (Lecommandoux et al, Macromol. 2001, 34, 9100-9111 ; Huesmann et al Macromol. 2014, 47, 928-936), thus PBLG likely undergoes similar conformational equilibrium, explaining bimodality in SEC.

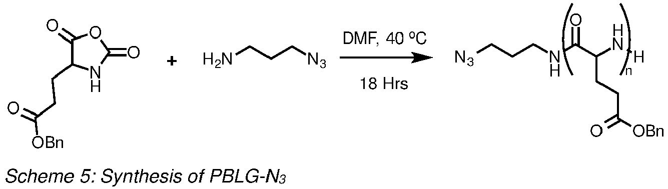

**Table A: PBLG-N₃ (^{a} determined by NMR, ^{b} determined by SEC)**

| **Entry** | **PBLGₙ-N₃** | **DP^{a}** | **Mn (g/mol)^{a}** | **Ð^{b}** |
|---|---|---|---|---|
| **1** | PBLG₂₀-N₃ | 20 | 4485 | 1.05 |
| **2** | PBLG₂₁-N₃ | 21 | 4704 | 1.13 |
| **3** | PBLG₂₆-N₃ | 26 | 5800 | 1.10 |
| **4** | PBLG₃₃-N₃ | 33 | 7333 | 1.10 |

### MeO-PEG₄₃-COOH .

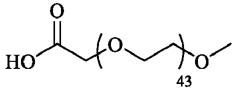

Polyethylene glycol monomethyl ether (2 kg·mol⁻¹) (5 g, 2.5 mmol), TEMPO (2 mg, 0.125 mmol) and KBr (30 mg, 0.25 mmol) were dissolved in 60 mL of DI H₂O. An 8% sodium hypochlorite solution (3 mmol sodium hypochlorite/mmol primary alcohol) was added, the pH of the reaction mixture was adjusted to pH ~10 and the reaction left to stir for 18 h. TLC showed 50% completion. EtOH (5 mL) was added and the pH reduced to pH -3, the mixture of product and starting material was extracted with DCM (×5) dried over MgSO₄, filtered, concentrated *in vacuo* and precipitated in Et₂O.

The precipitate was subjected to the above procedure with the same quantities of TEMPO, KBr, NaOCI and H₂O. The reaction was left for 48 h, TLC showed no starting material. EtOH (5 mL) was added and the pH reduced to pH -3, extracted with DCM (×5) dried over MgSO₄, filtered, concentrated *in vacuo* and precipitated in Et₂O to give the product MeO-PEG₄₃-COOH (4.3 g, 86%).

**¹H-NMR** (CDCl₃, 400 MHz): δ 4.15 (s, 2H, *CH*₂-COOH), 3.76 - 3.73 (m, 2H, PEG-CH₂), 3.69 - 3.62 (m, 168H, PEG-*C₂H₄*), 3.55 - 3.53 (m, 2H, PEG-CH₂), 3.37 (s, 3H, CH₃O).

### General method for the synthesis of Pico-PEG.

MeO-PEG-COOH (1.15 eq.) and linker (1 eq.) were dissolved dry DCM. EDC·HCl (2 eq) and DMAP (0.2 eq) were dissolved DCM and this was added to the reaction mixture and left to stir for 48 h. The reaction mixture was then precipitated in Et₂O, the precipitate was dried, re-dissolved in DCM and washed with brine. The product was extracted from brine with DCM (×5), dried over MgSO₄, filtered, concentrated *in vacuo,* dissolved in minimum acetone and precipitated in Et₂O to give the product Pico-PEG (79% - 99%).

### Pico-PEG

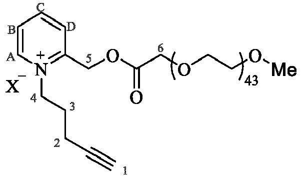

MeO-PEG₄₃-COOH (512 mg, 0.2604 mmol) and picolinium linker (58 mg, 0.2264 mmol) were dissolved 8 mL dry DCM. EDC·HCl (87 mg, 0.453 mmol) and DMAP (6 mg, 0.0453 mmol) were dissolved 2 mL dry DCM. (454 mg, 92%).

**¹H-NMR** (CDCl₃, 400 MHz): δ 10.25 (dd, *J* = 6.7, 1.1 Hz, 1H, **A**-aromatic CH), 8.49 (td, *J* = 7.9, 1.1 Hz, 1H, **C**-CH), 8.13 - 8.09 (m, 2H, **B**-CH, **D**-CH), 5.73 (s, 2H, **5**-CH₂), 5.21 (t, *J* = 7.7 Hz, 2H, **4**-CH₂), 4.33 (s, 2H, **6**-CH₂), 3.76 - 3.73 (m, 2H, PEG-CH₂), 3.60-3.70 (m, 168H, PEG-*C₂H₄*), 3.55 - 3.53 (m, 2H, PEG-CH₂), 3.37 (s, 3H, OCH₃), 2.52 (td, *J* = 6.4, 2.6 Hz, 2H, **2**-CH₂), 2.35-2.31 (m, 2H, **3**-CH₂), 2.06 (t, *J* = 2.6 Hz, 1H, 1-H).

### Block copolymer PEG₄₃-Pico-b-PBLG₂₁

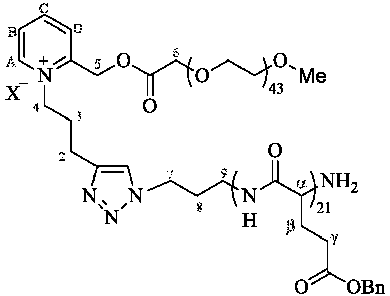

**Pent-4-yn-1-yl picolinium triflate** (232 mg, 0.1063 mmol) and PBLG-N₃ (200 mg, 42.5 µmol) were dissolved in dry THF (7 mL). Cul (8 mg, 42.5 µmol) and DIPEA (15 µL, 85 µmol) were added sequentially and the reaction mixture was left to stir under argon for 18 h. The reaction mixture was concentrated and purified by silica gel column chromatography (MeOH: DCM, v:v, 1:50 → 3:20). The product was re-dissolved in minimum DCM and precipitated in Et₂O. The precipitate was dried *in vacuo* to give the product (210 mg, 71%).

**¹H-NMR** (CDCl₃, 400 MHz;): δ 8.35 - 8.17 (m, 19H, amide-NH). 7.34 - 7.16 (m, 105H, **Bz-**aromatic-CH), 5.14 - 4.95 (m, 46H, **Bz**-CH₂, **4**-CH₂, **5**-CH₂), 3.98 - 3.80 (m, 23H, **α**-CH, **6-**CH₂), 3.79 - 3.46 (m, 174H, PEG-*C₂H₄*, **7**-CH₂), 3.38 (s, 3H, OCH₃), 3.31-3.35 (m, 2H, **9-**CH₂), 2.79 - 2.01 (broad m, 88H, **2**-CH₂, **3**-CH₂, **β**-CH₂, **γ**-CH₂), 1.86 - 1.82 (m, 2H, **8**-CH₂). SEC: Ð = 1.09.

### Synthesis of N-methyl quinolinium esters

### General procedure for the synthesis of quinoline aldehydes

The appropriate quinoline (1.0 eq.) was introduced to a suspension of selenium dioxide (1.3 eq.) in dioxane, and the mixture was heated at 80 °C for 3 h. After cooling the mixture to rt, the solid was filtered on celite, washed with EtOAc and concentrated under reduced pressure. The crude product was purified by column chromatography on silica gel (Cyclohexane/EtOAc: 4/1) to obtain the corresponding aldehyde as an orange-red solid.

### Quinoline-2-carbaldehyde

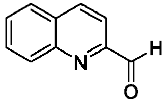

Following general procedure, 2 g (13.96 mmol) of quinaldine was reacted with 2.013 g (18.14 mmol) of selenium dioxide to obtain quinoline-2-carbaldehyde (2.01 g, 92%) **Chemical formula:** C₁₀H₇NO **Molecular Weight:** 157.17 g·mol⁻¹

**¹H NMR** (CDCl₃, 500 MHz): δ 10.24 (s, 1H), 8.32 (d, *J* = 8.5 *Hz,* 1H), 8.26 (d, *J* = 8.5 *Hz,* 1H), 8.04 (d, *J* = 8.5 *Hz,* 1H), 7.91 (d, *J* = 8 *Hz,* 1H), 7.83 (t, *J* = 10 *Hz,* 1H), 7.70 (t, *J* = 7 *Hz,* 1H).

**¹³C NMR** (CDCl₃, 125 MHz): δ 193.9, 152.8, 148.1, 137.5, 130.63, 130.6, 130.2, 129.4, 128.0, 117.5.

**MS (ESI):** *m*/*z* =158.1 [M+H]⁺, 189.9 (hemiacetal).

### General procedure for the synthesis of quinoline methyl-alcohols

To a solution of the corresponding aldehydes (1.0 eq.) in methanol at 0°C was added sodium borohydride (1.2 eq.). The mixture was stirred at rt for 1 h before being quenched with HCl 1M. Water was added and the product was extracted with DCM. The organic layer was washed twice with water and brine, dried over MgSO₄, filtered and concentrated under reduced pressure. The product obtained was used without further purification.

### Quinolin-2-ylmethanol

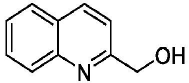

Following general procedure, 0.8 g (5.08 mmol) of quinolone carbaldehyde was reacted with 0.230 g (6.1 mmol) of sodium borohydride to obtain quinolin-2-ylmethanol (0.77 g, 95%).

**Chemical formula:** C₁₀H₉NO **Molecular Weight:** 159.19 g·mol⁻¹

¹H **NMR** (CDCl₃, 500 MHz): δ 8.14 (d, *J* = 8.5 *Hz,* 1H), 8.08 (d, *J* = 8.5 *Hz,* 1H), 7.82 (d, *J* = 8 *Hz,* 1H), 7.73 (m, 1H), 7.54 (m, 1H), 7.28 (d, *J* = 8.5*Hz,* 1H), 4.92 (s, 2H).

**¹³C NMR** (CDCl₃, 125M Hz): δ 159.1, 146.9, 137.0, 130.0, 128.8, 127.83, 127.80, 126.5, 118.5, 64.3.

### General procedure for the synthesis of brominated quinolines.

Aqueous HBr (49%) (10 mL) was directly poured into appropriate quinoline alcohols, and the mixture was heated up to reflux for 10 -12 h. Solvents were evaporated under reduced pressure and the crude was dried under vacuum in the presence of P₂O₅ to remove trace of water; Brown solid.

### 2-(Bromomethyl) quinoline hydrogen bromide

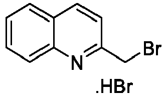

Following general procedure, 10 mL of aqueous hydrogen bromide was added to 1.0 g (6.0 mmol) of **alcohol** to obtain the desired halogenated compound (1.9 g, quant.). **Chemical Formula:** C₁₀H₈BrN **Molecular Weight:** 222.09 g·mol⁻¹

**¹H NMR** ((CD₃)₂SO, 500 MHz): δ: 9.18 (d, *J* = 8.27 *Hz,* 1 H,), 8.84 (d, *J* = 8.8 Hz,1 H), 8.26 (d, *J* = 10.2 *Hz,1* H), 8.04 (d, *J* = 8.7 *Hz,* 1 H), 7.85 (dd, *J* = 8.0,1.6 *Hz,* 1 H), 7.77 (m, 1 H) 5.12 (s, 1H).

**¹³C NMR** ((CD₃)₂SO, 500 MHz): δ 161.1, 159.5, 146.3, 138.8, 134.6, 132.5, 128.1, 127.5, 127.1, 67.1.

### General procedure for quinolinium PEG esters

The brominated quinoline (1.2 eq.) was dissolved into DMF in the presence of K₂CO₃ (2.5 eq.), MeO-PEG₄₃-COOH (1.0 eq.) was introduced into this solution & mixture was heated at 40 °C for 24 -32 h. After cooling the mixture to rt, brine solution was added to the reaction mixture washed with EtOAc (2 to 3 times) and concentrated under reduced pressure. The oily mass was further precipitated by diethyl ether to obtain the corresponding off-white solid.

### Quinoline 2-hydroxymethylene PEG ester

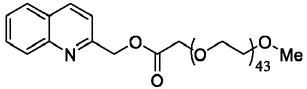

Following the general procedure, 2.0 g (1 mmol) of MeO-PEG₄₃-COOH was reacted with the bromo-compound (0.290 g , 1.3 mmol) in the presence of 0.414 g K₂CO₃ (3 mmol) to obtain the desired ester (1.7 g, 85%).

**¹H NMR** (CDCl₃, 500 MHz,): δ 8.22 (d, *J* = 8.2 *Hz* 1 H), 8.09 (d, *J* = 8.2 *Hz* 1 H), 7.85 (d, *J* = 8.5 *Hz* 1 H), 7.76 - 7.71 (dd, *J* = 9.4, 6.5 *Hz* 1 H), 7.60 - 7.54 (t, *J* = 8.24 *Hz* 1 H), 7.5 - 7.48 (d, *J* = 8.5 *Hz* 1H), 5.49 (s, 2H), 4.33 (s, 2H), 3.66 (s, 121 H, -CH₃ mPEG₂₀₀₀), 3.39 (s, 3H, -OCH₃ mPEG₂₀₀₀).

**¹³C NMR** (CDCl₃, 500 MHz): δ 174.4, 170.2, 155.6, 147.6, 137, 129.9, 129.2, 127.6, 126.7, 119.5, 71.9, 70.6 (m-PEG-CH₂), 69.7, 67.5, 59.

### General procedure for preparation of block co-polymers

Quinoline-PEG esters (1 eq) and pent-4-yn-1-yl trifluoromethanesulfonate (1.2 eq) were dissolved in DCM and stirred at room temperature for 72h. The mixture was evaporated after that these products used *in situ* as for preparation of block co polymers. Block copolymers were synthesized. Alkyne derivatives (1 eq.) and PBLG-N₃ (2.5) were dissolved, respectively, in dry THF (7 mL). Cul (1 eq.) and DIPEA (2 eq.) were added sequentially and the reaction mixture was left to stir under argon for 24 - 36 h. The reaction mixture was concentrated and redissolved in DCM, this solution was washed by aqueous solution of EDTA (10%) at least two times. The organic solution was dried over Na₂SO₄ and concentrated the mass under vacuum. The desired product was precipitated in diethyl ether. Off-white semi solid mass was obtained (60 -75%).

### Block co-polymer (Quino)

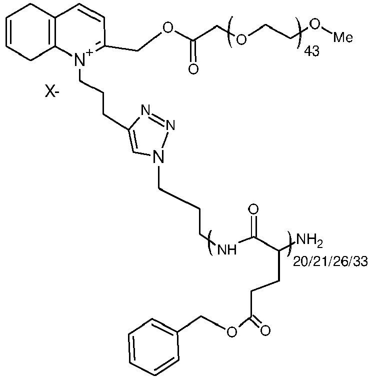

**100** (130 mg, 55 µmol) and PBLG₂₀-N₃/ PBLG₂₁-N₃ (100 mg, 22 µmol) were dissolved in dry THF (3.5 mL). Cul (8.3 mg, 4 µmol) and DIPEA (4.2 µL, 5 µmol) were added sequentially and the reaction mixture was left to stir under argon for 24 - 36 h. Semi-solid off-white color 97.5 mg (75/72%) compounds obtained respectively.

**¹H NMR** (CDCl₃ 500 MHz): δ

### Hydrophobically coated very small iron oxide nanoparticles (VSION)

Very small iron oxide NPs (VSIONs) of diameters around 4 nm were synthesized by the polyol route with slight modifications to control the nucleation-growth process through the water content of the solvent mixture, as reported in Hemery et al Inorganic Chemistry, 2017, 56(14), 8232-8243. Namely 1.082 g (4 mmol) of FeCl₃·6H₂O and 0.398 g (2 mmol) of FeCl₂·4H₂O were dissolved in 80 g of diethylene glycol (DEG). Separately, 0.64 g (16 mmol) of NaOH pearls was dissolved in 40 g of DEG. Both solutions were stirred overnight under nitrogen flux to prevent oxidation of the Fe(ll) species. The solutions were then mixed and stirred for 3 h, before heating the mixture at 210°C with an oil bath, reflux set-up and mechanical agitation. At first the set-up was open and a flux of nitrogen helped to remove traces of water. When a temperature of 210°C was reached the set-up was closed, and hot injection of 2.5 mL water (137 mmol) in the vessel through a septum led to a burst of nuclei. The formation of nanoparticles through forced hydrolysis pathway was carried out for 30 min, before letting the system cool down to RT by removing the heating plate. The black sediment was separated magnetically and washed 3× with a mixture of EtOH and EtOAc (1:1 v/v). Possibly present non-magnetic iron oxohydroxides were removed by treatment with 10% nitric acid. 8.6 g of Fe(NO₃)₃·9H₂O was then added to the solution as a strong oxidant by heating at 80 °C for 45 min while mechanically stirring. The solution then turned from clear black (magnetite Fe₃O₄) to red (maghemite γ-Fe₂O₃). The IONPs were then washed 2 times with acetone and 2 times with Et₂O before being dispersed in water. The morphology of nanoparticles was investigated by TEM and automated particle sizing led to a diameter range of 4.3±1.1 nm.

The commercial dispersing agent Beycostat^{™} NE (NB09, CECA, Puteaux, France) was used to coat the surface of the VSIONs and render them hydrophobic. This surfactant is a mixture of mono- and di-esters of phosphoric acid with poly(ethylene oxide)₉ nonylphenyl ether chains. To a mixture of 2.55 mL acidic aqueous VSION dispersion at 18.0 g/L iron oxide (45 mg solid content) and 235 mg of BNE (liquid), 6.5 mL of a 2 M HNO₃ solution was added and the reaction stirred vigorously (400 rpm) with a mechanical stirrer at 60 °C for ~15 minutes. The VSIONs were settled over a permanent magnet and the supernatant pipetted out. The BNE-coated VSIONs were washed 5× with 10 mL MeOH, before being dispersed in 2.5 mL of THF. The iron oxide content of this dispersion was estimated by dissolving 50 µL of suspension in 5 mL of HCl 5M with help of a sonication bath. The absorbance (optical density) at 350 nm was converted through predetermined calibration, OD_{350nm, 2mm}=0.5043×[Fe]_{mM}+0.0172, into a concentration of 0.91 g/L γ-Fe₂O₃ in THF. DLS measurement with multi-modal (CONTIN) analysis showed peaks at 7.5 and 37.5 nm.

### 2. Self-assembly process

**Preparation of self-assembled structures for morphology studies:**

### Nanoprecipitations of picolinium block co polymers:

Block copolymer **PEG₄₄-Pico-*b*-PBLG₁₇:** 900 µL of water was added fast (in one shot) to a solution (100 µL) of block co-polymer (1 mg) in DMSO. This was dialyzed (3.5 kDa cutoff membrane) against ultrapure water for 18 h. DLS measurement led to **Z-Avg** = 93 nm, **PDI** = 0.17. Micelle morphology was observed by TEM.

Here Z-Avg represented Z-averaged diameters (2 times *R*ₕ) determined by a 2^{nd} order Cumulant fit of the DLS curves.

Block copolymer **PEG₄₃-Pico1-*b*-PBLG₂₁:** 4.5 mL of water was added fast (in one shot) to a solution (500 µL) of block copolymer **Pico** (5 mg) in THF. It was dialyzed (3.5 kDa cutoff membrane) against ultrapure water for 18 h. DLS measurement led to **Z-Avg** = 143 nm, **PDI** = 0.105. Polymersomes where formed, as evidenced by MALS (ALV p-ratio = **1.1**), TEM and Cryo-TEM images.

2.67 mL of water was added quickly to a solution (330 µL) of block copolymer **Pico** (3.3 mg) in DMSO. The suspension was dialyzed (3.5 kDa) against ultrapure water for 18 h. DLS measurement led to **Z-Avg** = 293 nm, **PDI** = 0.261. Micelles were analyzed by TEM.

Nanoprecipitation with saline solution 2.7 mL of 0.9 % aq. NaCl was added quickly to a solution (300 µL) of block copolymer **Pico** (3 mg) in THF. The suspension was dialyzed (3.5 kDa cutoff membrane) against 0.9 % aq. NaCl for 18 h. **Z-Avg** = 120 nm, **PDI** = 0.118. Polymersomes were analyzed by TEM and MALS (ALV p-ratio = **1.2**).

Nanoprecipitation with hydrophobic γ-Fe₂O₃ VSIONs A suspension (6.6 µL, 5% FWR relatively to polymer) of Beycostat coated 4.3±1.1 nm diameter γ-Fe₂O₃ VSIONs was added to a solution (200 µL) of block copolymer **Pico** (2 mg) in THF and nanoprecipitation occurred by fast addition of water (1,7 mL). This suspension was kept into a semi-permeable membrane tube (3.5 kDa cutoff) & dialyzed against ultrapure water for 18 h. The nanoparticles were analyzed by DLS: **Z-Avg** = 129 nm, **PDI** = 0.273. Polymersome morphology with iron oxide nanoparticles buried in the hydrophobic membranes were observed by TEM and cryo-TEM.

Nanoprecipitation with hydrophobic γ-Fe₂O₃ NPs in saline solution A suspension (15 µL, 10% FWR relatively to polymer) of Beycostat coated 4.3±1.1 nm diameter γ-Fe₂O₃ VSIONs was added to a solution (220 µL) of block copolymer **Pico** (2.2 mg) in THF, then 0.9% aq. NaCl (2 mL) solution was added fast into it. This suspension was dialyzed (3.5 kDa cutoff) against 0.9% aq. NaCl for 18 h. DLS analysis led to **Z-Avg.** = 142 nm, **PDI** = 0.201. Polymersome formation was ascertained by MALS (ALV p-ratio = **1.0**) and iron oxide nanoparticles buried in their hydrophobic membrane were observed by TEM.

### Nanoprecipitations of quinolinium block co polymers:

Block copolymer **Quino** (1 mg) dissolved in THF (100 µL) and (900 µL) of 0.9% aq. NaCl was added promptly into it for nanoprecipitation. This solution was put in a dialysis bag (15 kDa cutoff) and dialyzed against 0.9% aq. NaCl for 24 h. DLS measurement led to **Z-Avg** = 88 nm, **PDI** = 0.10. TEM pictures suggested the formation of polymersomes.

900 µL DI water was added promptly into the solution of Block copolymer **Qino** (1 mg) in THF (100 µL) for nanoprecipitation. This solution was put in dialysis bag (15 kDa cutoff) and dialyzed against DI water for 18 h. Hydrodynamic size was measured by DLS: **Z-Avg** = 73 nm, **PDI** = 0.231.

Block copolymer **Quino** (1 mg/mL) dissolved in THF (100 µL) was added into (900 µL) DI water for nanoprecipitation. This solution was put in dialysis bag (15 kDa cutoff) and dialyzed against ultrapure water for 18 h. DLS measurement led to **Z-Avg** = 59 nm, **PDI** = 0.138. The TEM picture showed small vesicles that might rather be micelles, showing that the addition order is important (with **Quino** copolymer; vesicles are more likely formed when the aqueous solution is poured into organic polymer solution rather than with the reverse addition order).

Nanoprecipitation in saline Block copolymer **Quino** (1.5 mg) was dissolved in THF (100 µL) and (900 µL) of 0.9% aq. NaCl was added quickly into it for nanoprecipitation. This suspension was put in a dialysis bag (15 kDa cutoff) and was dialyzed against 0.9% aq. NaCl for 24 h. DLS measurement yielded a hydrodynamic diameter of **Z-Avg** = 107 nm, **PDI** = 0.130. The morphology was characteristic of polymersomes according to TEM and MALS (ALV p-ratio = **1.0**).

### Nanoprecipitation with hydrophobic γ-Fe₂O₃ NPs in saline

A suspension (23.4 µL, 5% FWR relatively to polymer) of Beycostat-coated 4.3±1.1 nm diameter γ-Fe₂O₃ VSIONs was added to a solution (100 µL) of block copolymer **Quino** (1.5 mg) in THF. Then 0.9% aq. NaCl (0.9 mL) was added quickly to the solution. The suspension was dialyzed (3.5 kDa cutoff) against saline for 18 h. Hydrodynamic diameter measured by DLS was **Z-Avg** = 130 nm, **PDI** = 0.120. Morphology according to TEM images consisted of polymersomes loaded with iron oxide NPs in their membrane.

### Characterization

### GPC

Gel permeation chromatography (GPC) also called size exclusion chromatography (SEC) was performed on an Ultimate 3000 system from Thermo Fisher scientific equipped with a differential refractive index detector (dRI) detector from Wyatt Technology corporation (Santa Barbara CA, USA), using DMF with LiBr (1 g/L) as the eluent at a flowrate of 0.8 mL/min. The column set consisted of two KD-803 Shodex^{™} gel columns (300 × 8 mm) (exclusion limits 1-50 kDa), maintained at 50°C. Weight-average molar massed (M_{w}) and mass dispersities (D) were calculated with ASTRA 7.1.0 software from the chromatograms calibrated using a series of polystyrene standards.

### DLS

The hydrodynamic radii and size distribution of the self-assembled copolymers were measured at by dynamic light scattering (DLS) on a Zetasizer^{™}, nano ZS instrument (Malvern company, UK) equipped with a 633 nm helium-neon laser using back-scattering detection at fixed 173° scattering angle on samples equilibrated at 25°C. The measured hydrodynamic sizes of the vesicles as well as the PDI values were obtained by the classical 2^{nd} order Cumulant fitting procedure of the scattered intensity correlograms (T = 25 °C, 13 runs of 10 - 15 s duration times, measurements in triplicates to derive a standard deviation of all values), while the derived countrate in kcps (mean scattered intensity normalized by the attenuator transmission factor) was used as an indication of the concentration of scattering objects (at a constant aggregation number or size of the scattering objects).

### SLS

Multi-angle light scattering measurements (MALS) were performed using an ALV/CG6-8F goniometer, with a 35-mW red helium-neon linearly polarized laser (λ = 632.8 nm) and an ALV/LSE-5004 multiple tau digital correlator at 20°C. The data were acquired with the ALV correlator software, the counting time was typically 15 s at each different scattering angles ranging from 30° to 150°, in 5 increments. The radius of gyration (*R*_{g}) was determined from a Guinier plot resulting from the measurement of the average scattered intensity at the same angles. In addition, the hydrodynamic diameter (*R*ₕ) was obtained by multi-angle measurement of the main decay rate Γ (by 2^{nd} order Cumulant fitting of the correlogram) at each angle and plot of Γ as a function of the square of the scattering vector q (defined by the classical formula as a function of the scattering angle, refraction index of water and wavelength of light), in order to derive the translation diffusion constant of the objects and convert it into a hydrodynamic radius using the Stokes-Einstein formula.

### TEM

Transmission electron microscopy (TEM) images were recorded using a FEI Tecnai microscope working at 120 kV equipped with an OSIS Megaview II megapixel camera. TEM samples were prepared by depositing 5 µL of the sample solution (1 mg/mL) onto an Agar Scientific Formvar^{™}/carbon 200 mesh copper grid for 1 min, blotted to remove the excess and followed by staining with a 1.5% uranyl acetate solution for 30 s.

### 3. Drug encapsulation and release

### Preparation of drug-loaded vesicles

A solution of doxorubicin hydrochloride (Dox·HCl) in 0.9 % of aq. NaCl solution (1 mM) was prepared. It was added rapidly (900 µL) to a copolymer (**Quino,** 2.1 mg) solution in THF. Solvent and excess of doxorubicin was removed by dialysis against 0.9 % of aq. NaCl solution (for 2 mL of solution, 2 × 3 L brine exchange, using a 15 kDa cutoff dialysis membrane). DLS measurements yielded **Z-Avg** = 93 nm, **PDI** = 0.23.

### Nanoprecipitation with hydrophobic γ-Fe₂O₃ NPs

A suspension (117 µL, 20% feed weight ratio (FWR) iron oxide relatively to polymer, e.g. 0.42 mg) of Beycostat^{™}-coated 4.3±1.1 nm diameter γ-Fe₂O₃ VSIONs was added to a solution (100 µL) of block copolymer **Quino** (2.1 mg) in THF and a solution of Dox·HCl in 0.9 % of aq. NaCl solution (1 mM, 783 µL) was added quickly into it. The suspension was dialyzed (15 kDa cutoff) against 0.9 % of aq. NaCl solution for 24 h. DLS measurements led to **Z-Avg** = 141 nm, **PDI** = 0.25. Morphology characteristic of polymersomes was observed by TEM.

### Fluorimeter measurements

Release of the encapsulated compound: The amount of released dye was followed at RT by fluorescence spectroscopy (Hitachi F7000 spectrometer). Chemically reduced or irradiated samples were placed in low volume Quartz cuvettes (50 µL) and directly analyzed. The *λₑₓ* excitation wavelength was set at 480 nm and the emission *λₑₘ* spectra were recorded from 490 to 720 nm. The instrument was used in scan mode, with excitation slit set to 5 nm and emission slit set to 5 nm.

### In vitro cytotoxicity studies

The cytotoxicity of **Pico-Ps** and **Pico-Ps(Fe)** as well as of **Quino-Ps** and **Quino-Ps(Fe)** were evaluated on cancer and healthy cells by the colorimetric MTT assay using 3-(4,5-dimethylthiazolyl-2)-2,5-diphenyltetrazolium bromide as reagent to test the mitochondrial activity of living cells. The cytotoxicity of **Pico-Ps** and **Pico-Ps(Fe)** was determined on BWGT3 tumoral hepatocytes and TIB75 healthy hepatocytes chosen as reference cells. Cells were seeded at 2 × 10⁴ cells per well in 100 µL of culture medium and were incubated for 24 h at 37 °C in 5% CO₂-humified atmosphere. **Pico-Ps(Fe)** and **Pico-Ps** polymersomes were incubated for 1 h in serum-free RPMI-1640 culture medium at various concentrations (3 wells per condition, repeated twice). Cells were washed with phosphate buffered saline (PBS) and incubated during 1 h in full culture medium. Absorbance at 480 nm was measured in each well. The viability was calculated by reference to the cells without addition of polymersomes. The half maximum inhibitory concentration leading to 50% cell-viability (IC50) was then calculated by nonlinear regression analysis using GraphPad Prism 6 software (La Jolla, CA, USA).

Five different concentrations (0, 50, 100, 300, and 500 µg/mL) of **Pico-Ps(Fe)** and **Pico-Ps** were prepared by dilution of aliquot samples with cell media, respectively. **Pico-Ps(Fe)** appeared invariably (but only slightly) more toxic than **Pico-Ps** after 24 h of incubation with TIB75 cells. In contrast, cell-cultures showed almost negligible cytotoxicity of polymersomes on BWTG3 cells even at higher concentrations (up to 500 µg/mL).

Also, the cytotoxicity of quinolinium-derived Ps, **Quino-Ps** and **Quino-Ps(Fe)** was studied on epithelial cell line BNL 1ME A.7R.1 (American Type Culture Collection TIB-75). Cells were cultured in 25 mL of Dulbecco's Modified Eagle Medium (DMEM), with a high glucose content (4.5 g/L, GlutaMAX), and were supplemented with 10% fetal calf serum (FCS) and 1% penicillin and streptomycin. Cells were placed in 75 cm² flasks incubated at 37 °C with 5% CO₂ and sub-cultured by Trypsin-0.05% EDTA (4 mL). Cells were plated at a cell density of 2.0 × 10⁵ cells per well in a 96-well (100 µL/well) plate and incubated for 24 h at 37 °C under 5% CO₂ atmosphere before treatment. After incubation, buffer was replaced by freshly prepared brine (0.9% v/w) and 100 µL of **Quino-Ps** and **Quino-Ps(Fe)** were deposited, respectively in three wells, in a triplicated analysis. Cells were incubated further at 37 °C under 5% CO₂ atmosphere. After incubation with the polymersomes, the medium was removed and replaced by 100 µL of the MTT reagent (0.5 mg / mL prepared in the culture medium). After 4 h incubation at 37 °C the reagent was carefully removed and 100 µL of DMSO was added to each well in order to dissolve the colored formazan product. Then the plates were shaken on an orbital shaker for 10-15 minute for dissolving the formazan, and the absorbance was measured by using microplate reader (Infinite^{®} 200 PRO series Tecan) at 562 nm (three replicates were read for each samples). The relative cell viability (%, n = 3) was calculated (for both **Quino-Ps** and **Quino-Ps (Fe))** as the percentage of living cells of the treated wells compared to the untreated cells. As before, cell-cultures showed almost negligible cytotoxicity at incubated concentration up to 500 µg/mL for the quinolinium probe based polymersomes, with or without VSION loading.

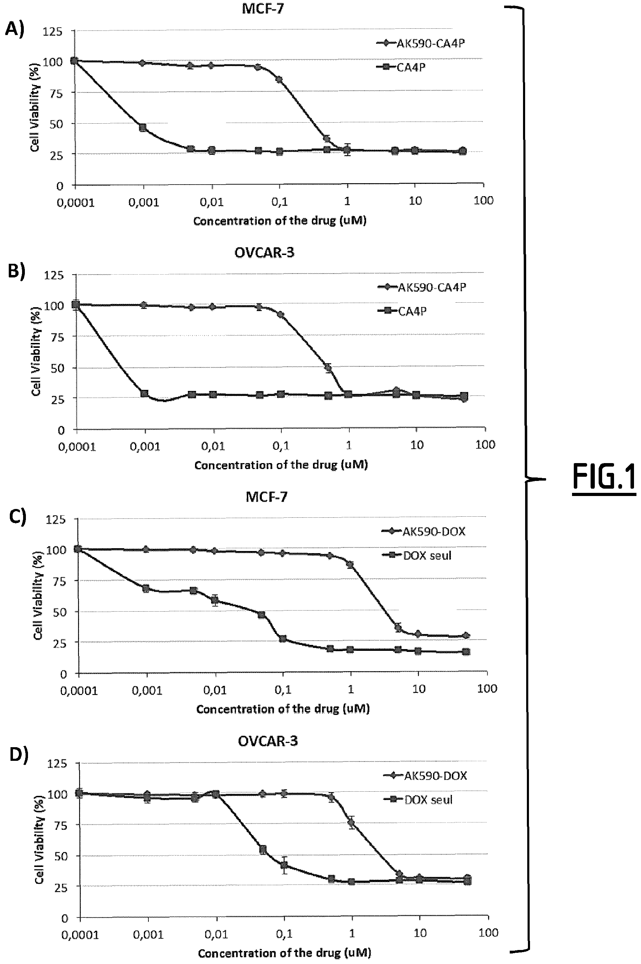

In another series of experiments, the cytotoxicity of combretastatin and doxorubicin-filled **Quino**-Ps were compared on MCF-7 human breast cancer and Ovar-3 carcinoma cell lines (Figure 1). As it can be seen, the encapsulated drugs preserve some of their original biological activity / toxicity, although exhibiting 100-1000 times larger values of IC50 of these two APIs compared to the reference (non-encapsulated) compounds. As no leaching of the encapsulated product was evidenced under similar conditions, the reason of the residual biological activity can be the adsorption of a small fraction of the active compound at the surface of the polymersomes in addition to the main part encapsulated inside their lumen. In both cases, these results demonstrate the efficiency of encapsulation in polymersomes to protect healthy tissues as long as they are not subjected to the external or endogenous signal triggering API release.

### In vivo biodistribution of the prepared (unloaded polymersomes

Proton relaxivities of polymersome **Pico-Ps(Fe)** were measured in saline (9% v/w NaCl). Samples were concentrated to reach 1.0 mM_{Fe} in equivalent iron concentrations (i.e. 0.08 g/L iron oxide) and a series of dilutions were prepared (*c*_{Fe} = 1.0, 0.5, 0.2, 0.1, and 0.02 mM). The longitudinal relaxation time (T₁) of water protons was measured by saturationrecovery spin-echo sequence (RARE images; echo time TE = 13 ms; 10 repetition times TR = 15 s, 8 s, 3 s, 1.2 s, 0.800 s, 0.594 s, 0.300 s, 0.144 s, 0.050 s, 0.033 s, repetition time TR = 5000 ms, 1500 ms, echo time TE = 13 ms) on a 300 MHz MRI scanner (7 T, 30WB, Bruker Biospin) at 25 °C, while transversal (T₂) relaxation times were measured by a multi spin-echo train acquisition MSME images (TR/TE = 15 s / 11 ms, 32 echos; 11 to 32 × 11 ms). T₂* relaxation times were measured by using a multi echo gradient echo (GRE) sequence (24 echoes TE = 4 ms, TR= 5 s).

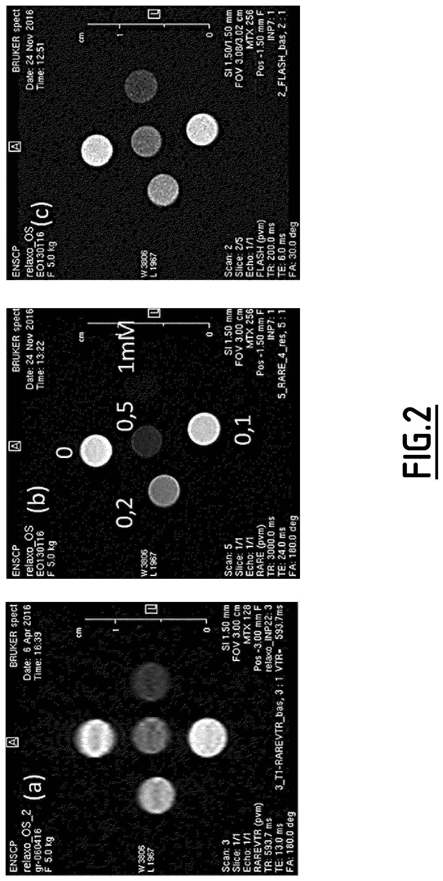

Fields of view of 3 × 3cm², a matrix size of 128 × 64 and a slice with a thickness of 1.5 mm were used for T₁ and T₂ maps. For T₂* mapping, a field of view of 3 × 3 cm², a matrix of 256 × 192 and a slice with a thickness of 1.5 mm were used. The relaxation rates 1/Tᵢ were plotted against iron concentration, and the corresponding longitudinal and transverse relaxivities rᵢ were obtained by linear regression 1/Tᵢ = (1/Tᵢ)_{buffer}+ rᵢ C_{Fe} (Figure 2). **Pico-Ps(Fe)** exhibits r₁ = 0.5 s⁻¹·mM_{Fe}⁻¹ in deionized water and r₁ = 0.1 s⁻¹·mM_{Fe}⁻¹ in physiological saline, respectively as well as r₂=137 s⁻¹·mM_{Fe}⁻¹ in DI water and r₂ = 43 s⁻¹·mM_{Fe}⁻¹ in NaCl, respectively; r₂* = 119 s⁻¹·mM_{Fe}⁻¹ in DI water and r₂* = 103 s⁻¹·mM_{Fe}⁻¹ in NaCl were calculated respectively. The low values of r₁ are ascribed to the hydrophobic environment of the VSIONs embedded in the polymersome membranes, impeding any direct "internal sphere" relaxation mechanism of water protons. On the contrary r₂ values are pretty high and close to values of commercial MRI contrast agents such as Resovist^{®}/Cliavist^{®} (discontinued) or Feraheme^{®} (currently on market [Yi-Xiang J Wang, Current status of superparamagnetic iron oxide contrast agents for liver magnetic resonance imaging, World J Gastroenterol. 2015, 21, 13400-13402]) due to the "external sphere" mechanism (i.e. dipolar effect of VSION magnetization working at distance on the water protons) that is enhanced by their clustering within the membranes. Noteworthy, the (r₂*/r₁) values were high enough to use these magnetic polymer vesicles as negative MRI contrast agents resulting into the "darkening" of specific locations of dynamic susceptibility-enhanced (DSC-MRI) (T₂*) weighted-images in tissues where they accumulate (Figure 2).

Likewise, the longitudinal and transversal relaxations of **Quino-Ps(Fe)** were measured by preparing a range of 0.1, 0.2, 0.5 and 1 mM equivalent iron concentrations of suspensions and placing the 5 samples in the MRI spectrometer.

In order to evaluate the contrast agent capacity / efficacy of **Quino-Ps(Fe) polymersomes,** a sample was compared to a commercial iron oxide contrast agent (Cliavist) chosen as reliable T₂ contrast reference. The measured relaxivity (r₁, r₂) values of **Quino-Ps(Fe)** *vs*. Cliavist^{®} (same as Resovist^{®}) are summarized in Table 1.

**Table 1. Comparison between the relaxivity values r₁ and r₂ of iron-based contrast agents, Cliavist^{®} (Resovist^{®}) taken as gold standard, and Quino-Ps(Fe) polymersomes. R² are the coefficients of determination of the linear fits.**

| **Relaxivity (mM⁻¹s⁻¹)** | **Cliavist (Resovist)** | **Quino-Ps(Fe)** |
|---|---|---|
| r₁ (R²) | 2.206 (0.99) | 0.38 (0.99) |
| r₂ (R²) | 142.4 (0.98) | 108 (0.90) |
| r₂/r₁ | 67 | 284 |

Although the longitudinal and transverse relaxivity values *r₁* and r₂ of **Quino-Ps(Fe)** were somewhat weaker than that of the Cliavist^{®} / Resovist ^{®} due to the very small core size of VSIONs, the combination of their local hydrophobic environment and clustered state in the membranes lead to a **Quino-Ps(Fe)** sample that is expected to show superior T₂ MRI contrast agent proprieties *in vivo* as the *r₂*/*r₁* ratio is as high as 284.

### MRI biodistribution study on animal model

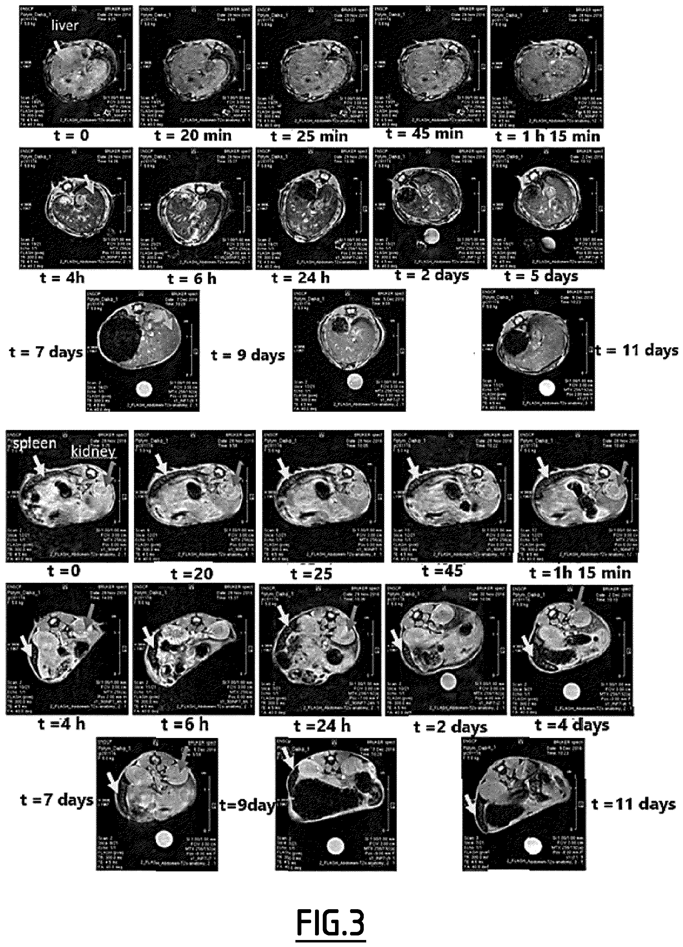

For recording the dynamic bio-distribution of **Pico-Ps(Fe)** in mice under *in vivo* conditions, a specific protocol, developed by the MR imaging facility, was applied (G. Ramniceanu, B-T. Doan et al, Delayed hepatic uptake of multi-phosphonic acid polyethylene glycol) coated iron oxide measured by real-time Magnetic Resonance Imaging RSC Advances 2016, 6, 63788-63800). After anesthesia by using 1.5 % isoflurane gas in air / O₂ mixture (0.5 L / min and 0.2 L / min, respectively), a 30G catheter was introduced in the caudal vein, and the mouse was installed in a whole-body 40 mm inner diameter RF birdcage coil (Bruker) with a tube of water as a phantom signal reference. A Dynamic Susceptibility Contrast (DSC) imaging methodology was used for the visualization / capture of **Pico-Ps(Fe)** magnetic polymersomes by the organs. IntraGateFLASH images were recorded to suppress breath motion artefacts in a kinetic way with 3 min duration and with adapted time resolution according to the uptake and clearance of the **Pico-Ps(Fe)** up to 11 days: TR/TE = 90 ms / 3 ms, 52 repetitions. Field of view of 3 × 3 cm², a matrix size of 256 × 256, for a 117 µm in-plane resolution, and 5 slices distant of 3 mm with a thickness of 1 mm were used. After 3 reference image acquisitions, 100 µL of **Pico-Ps(Fe)** (c = 1.42 mM of Fe in 0.108 M NaCl 0.9% saline) were injected. The biodistribution and clearance were monitored after injection with 3 min time resolution for 1 h, 4 h, 6 h than daily up to 11 days (Figure 3).

### Biodistribution study of Quino-Ps(Fe) in mice by MRI

The study of the biodistribution of **Quino-Ps(Fe)** was performed on Balb/c wild type female mice (n = 6), aged between 8 to 10 weeks on average. The system was equipped with a temperature-controller set-up, a pneumatic pressure sensor for breathing control, and a face-mask allowing a volatile anesthesia for *in vivo* imaging of small animals. Mice were anesthetized with 4% isoflurane and kept asleep in the restraining bed during the experiment at a dose of 1.5% and under an oxygen-air mixture (30:70, 0.2 L min⁻¹). The respiration was monitored throughout the experiments using a plastic sensor positioned at the chest of the mice and a physiological monitor to monitor the respiration. In parallel, a 5 mm saline tube was introduced to each mouse as a reference "phantom" for the quantification of the MRI signal. The mouse was positioned in a radiofrequency coil and inserted into the MRI spectrometer. A bolus of 200 µL of **Quino-Ps(Fe)** (c = 2.5 mM of Fe) suspension corresponding to 16.6 µmol Fe / kg dose) was injected intravenously (caudal vein) *via* a 0.38 mm internal and 1.09 mm external diameter catheter, adapted to a 30 G needle.

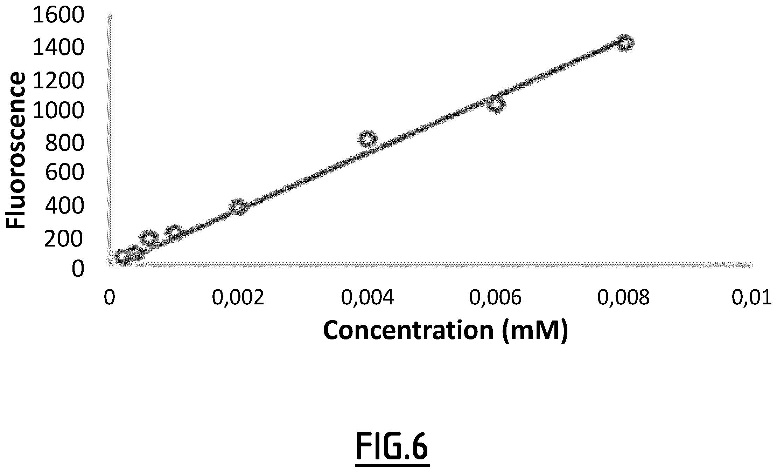

The kinetic monitoring of the biodistribution in the liver, spleen and kidneys i.e. the capture of the **Quino-Ps(Fe)** by the reticulo-endothelial system (RES) allowed evaluating their circulation time (Figure 6). For the MRI analysis, a flash gradient dynamic susceptibility contrast (DSC) echo sequence was used, according to Hoa *et al.*( Sauramps Medica, 2008, Montpellier, France) with the Intragate (Bruker) method that detects the respiratory and cardiac cycles of the mouse during the sequence. A post-processing operation allowed the elimination of the movements and the reconstruction of images without artefacts. This low 30° pulse angle sequence made possible to use short echo (TE = 3 ms) and repetition times (TR = 90 ms) while maintaining a weighted T₂* value. The short repetition rate thus allowed high temporal resolution (of 1 min) with good spatial resolution (117 × 117 µm² /pixel) in good sensitivity.

The accumulation of **Quino-Ps(Fe)** in the organs resulted in decreased MRI signal in T₂* weighted sequence, and thus decreased proton T₂* relaxation time of the surrounding water. The FLASH-Anatomy sequence set (in the Paravision 5.1 software) allowed the selection of five anatomical slices containing the liver, spleen and kidneys. This sequence used a trigger module to synchronize the acquisition with the breathing trays of the animal, and avoid motion artefacts: IntraGateFLASH sequences of the DSC imaging method were applied to these five slices for 3 min. The study was followed for 1 h (1 scan FLASH IG / 10 mn), thus points were acquired after 3 h, 6 h and were more spaced for two weeks. *In vivo* polymersome tracking was performed by 7T MRI bio-imaging. To follow the kinetics of the capture and clearance of the **Quino-Ps(Fe)** in the target organs, regions of interest were manually designed on the MRI images (Figure 6). By this way it was possible to quantify the retention, remanence and elimination in various organs, characteristics for the biodistribution of the **Quino-Ps(Fe)** imaging agent *in vivo (*G. Ramniceanu, B-T. Doan et al RSC Advances 2016, 6, 63788-63800).

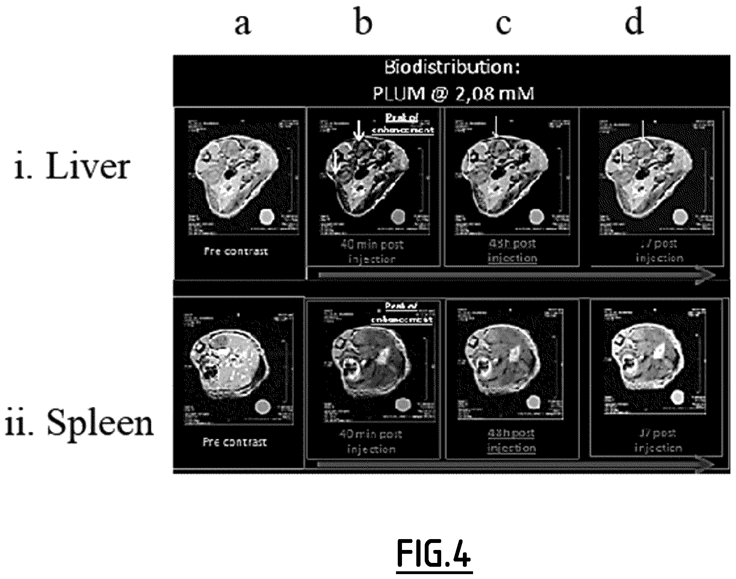

Before injection of the bolus of **Quino-Ps(Fe)** (20% FWR iron oxide), the liver appears as an "iso-signal" on the IntraGateFLASH images with respect to the arteries (Figure 4). During the hepatic capture of the contrast product, one can notice a hyposignal on the liver MRI sections. Looking at the kidneys (i), the MRI contrast seems visually invariable (weak build-up). Considering the spleen (ii), the picture shows significant hyposignal that persists up to 2 weeks after the injection of the nanoparticles.

As demonstrated by the results reported above, the prepared magnetic polymersomes fulfill the stringent requirements of biocompatibility and stealth properties, parameters that influence diffusion properties and stability under physiological conditions as well as some toxicity issues.

### Activation of polymersomes by local electron-transfer (ET) using different trigger signals (i.e. reducing agents)

The liberation of doxorubicin was followed by monitoring the fluorescence. Fluorescence spectra were collected by excitation at λₑₓ = 470 nm wavelength and reading at λₑₘ = 600 nm. In order to quantify the encapsulated drug, a method for the quantitative liberation was needed. Different methods, such as solvolysis, photolysis, addition of redox agents (sodium dithionite, mercaptoethanol, sodium thiosulfate, glutathione) were tested and are discussed as follows.

### Glutathione (GSH)-triggered drug release from Quino-Ps-DOX

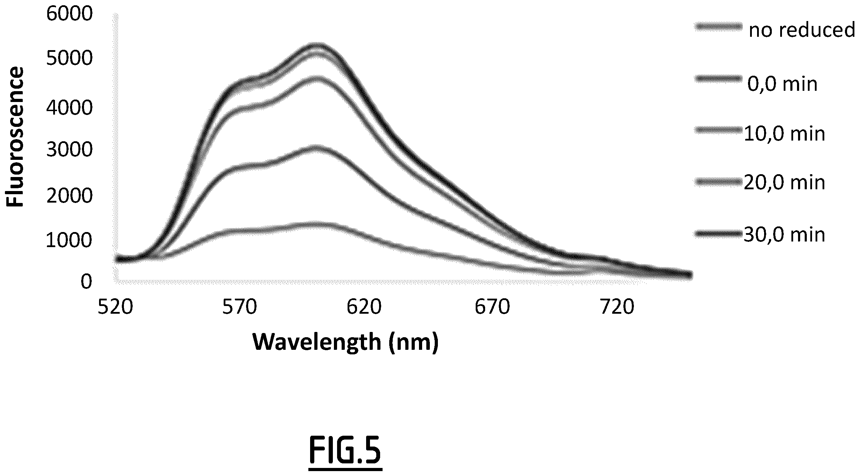

In parallel, **Quino-Ps-DOX** (50 µL) was treated with the natural enzyme glutathione (GSH) in its reduced form, 1 µL at *c* = 10 mM in brine (0.9% (v/w). The concentration of the GSH was set to 10 mM in order to be compatible with the highest intracellular level (Figure 5).

In the control (blank) experiment, doxorubicin (Dox) solution (50 µL *c* = 1.0 mmol in brine 0.9% (v/w)) was treated with the GSH solution (reduced form, 1.0 µL *c* = 10 mM in brine 0.9% (v/w)): no change in the fluorescence intensity of doxorubicin was observed, indicating that the observed fluorescence variation is not the consequence of a side reaction of GSH with Dox, and also that Dox appears stable under the experimental conditions.

The amount of the liberated drug was quantified by fluorescence (*λ*ₑₓ = 470 nm and *λ*ₑₘ = 600 nm), by using a calibration curve (Figure 6) (y= 1.79x ×10⁵ (R² = 0.9911)). According to this calibration curve, the cumulative liberation of Dox (after "30 min") was approximately 58% by considering that initially 1.0 mM concentration (approx. 544 µg) of drug was used for encapsulation.

Using the calibration curve, the amount of the released drug was calculated as 321 µg. Based on the released amount of drug, a drug loading content (rate of Dox weight over copolymer carrier weight) of 42% and a drug entrapment efficiency (rate of Dox encapsulated over Dox initially added at preparation) of 16% were obtained.

Samples were activated by X / gamma rays as well. Fast fragmentation was observed by using beam of the ID17 biomedical beamline at ESRF synchrotron (Grenoble, France) (conditions: Cuvette: quartz, d (light path): 1 mm, Beam: 120 keV, Bunch length: 48 ps, Bunch repetition rate 5.68 MHz, Dose: 30 Gy). For gamma rays, a conventional medical irradiator in a hospital was used.

### Stability on storage:

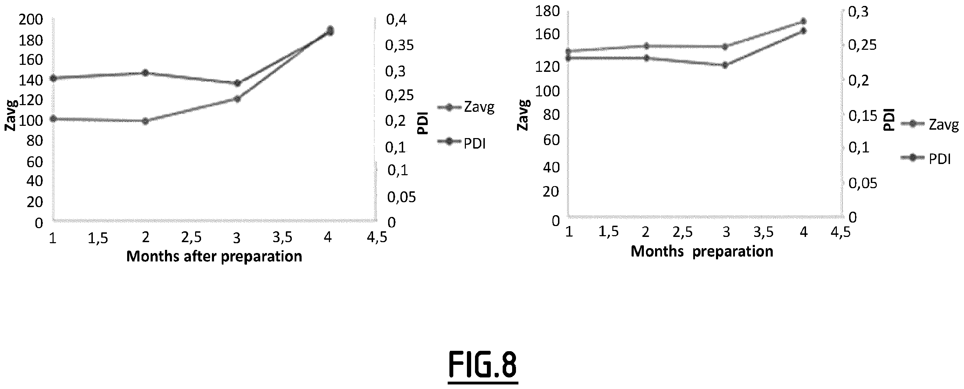

Samples were stored at 4 °C. An aliquot of each sample (1.0 mL) was analyzed by DLS over 3 - 4 months repeatedly. Both **Quino-Ps** (Figure 8A) and **Quino-Ps(Fe)** (Figure 8B) samples appeared stable under the storage conditions (at 4°C) for 4 months, although a slight Zavg increase with small variation in PDI was observed. Same long term shelfstability was observed for **Pico-Ps** and **Pico-Ps(Fe)** as summarized in Table 2.

**Table 2. Long term stability study of Pico-Ps and Pico-Ps(Fe) samples as assessed by DLS up to one year. Z-Ave diameter and PDI correspond to 2^{nd} order Cumulant analysis of the correlograms, and PDI width is the characteristic broadness of the size distribution calculated by multiplying Z-A ve by the square root of the PDI value. The derived countrate (DCR) is the normalized scattered intensity, which gives indication on the state of aggregation (it increases when colloidal particles are clustered together, or decreases if they are degraded or settle at the bottom of the vial).**

| Sample Name | Measurement Date and Time | *T* °C | Derived Count Rate (kcps) | Z-Ave diameter (nm) | PDI Width (nm) | PDI |
|---|---|---|---|---|---|---|
| NP5_Pico-Ps Reproduced | 09/09/2016 15:48 | 25 | 6064.5 | 120.1 | 41.3 | 0.118 |
| NP5_Pico-Ps Reproduced (Fitrated 0.2 µm) | 09/09/2016 11:55 | 25 | 5715.1 | 118.4 | 40.4 | 0.116 |
| NP5 Pico-Ps (Sterile, in NaCl 9 g/L) | 19/10/2017 15:08 | 25 | 5346.3 | 114.4 | 38.4 | 0.113 |
| NP5 Pico-Ps (NaCl 9 g/L) (Synchrotron irradiation) | 27/11/2017 19:18 | 25 | 5323 | 116.8 | 41.8 | 0.128 |
| NP6 Pico-Ps | 04/11/2016 11:13 | 26.2 | 7985.5 | 145.3 | 85.6 | 0.347 |
| NP6 Pico-Ps (Filtrated 0.2 µm) | 04/11/2016 12:16 | 25 | 4333.5 | 105.1 | 43.2 | 0.169 |
| INP7 Pico-Ps(Fe) | 04/11/2016 11:26 | 25 | 18581.3 | 155 | 68.7 | 0.197 |
| INP7 Pico-Ps(Fe) (Filtrated 0.2 µm) | 04/11/2016 12:25 | 25 | 15157.9 | 135.5 | 48.4 | 0.128 |
| INP7_ Pico-Ps(Fe) (Conventional irradiation) | 28/11/2016 18:51 | 25 | 16244.7 | 146.9 | 57.4 | 0.153 |
| INP7_ Pico-Ps(Fe) (Synchrotron irradiation) | 08/12/2016 17:19 | 25 | 13346.4 | 139 | 55.1 | 0.157 |
| INP7 Pico-Ps(Fe) 1.42mM Fe NaCl 9q/L | 19/10/2017 14:50 | 25 | 13887.5 | 143 | 58.7 | 0.168 |
| INP7 Pico-Ps(Fe) 1.42mM Fe NaCl 9g/L (2^{nd} synchrotron irradiation) | 27/11/2017 19:50 | 25 | 14744 | 143.8 | 53.4 | 0.138 |

### Non-covalent modification of the polymersome bi-layer by plasmonic gold nanoparticles (GPNP6P) for fluorescence tracking, cancer diagnosis and photothermal therapy.

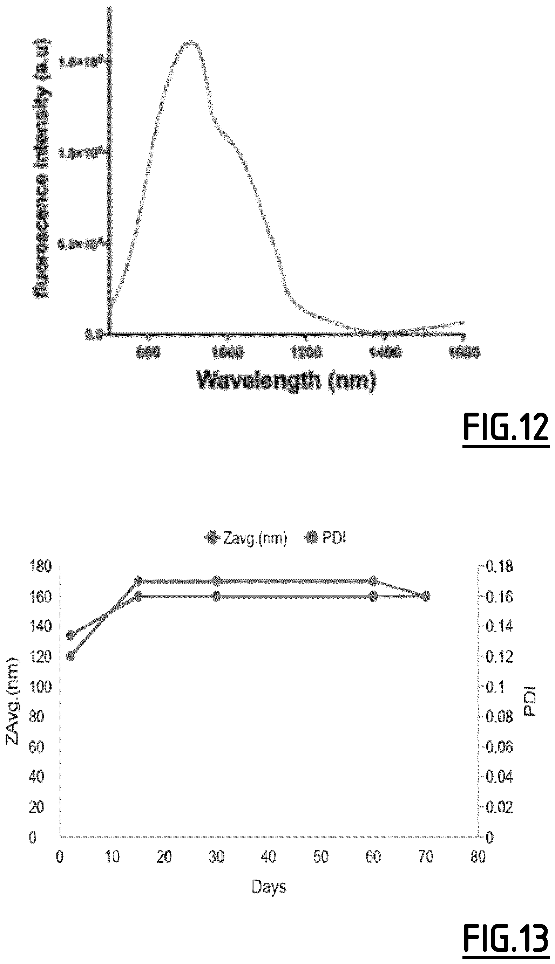

Gold nanoparticles (GNPs) were prepared according to the method disclosed by Y. Chen et al Nano Lett. 2017, 17, 6330-6334 (DOI: 10.1021/acs.nanolett.7b03070) and were mixed with the picolinium-derived block co-polymer described above in THF. The nanoprecipitation was realized by following the above nanoprecipitation procedure and the samples were analyzed by UV- Vis spectrophotometer. Results are illustrated in Figure 12) and by DLS (Table 3 below). The hydrolytic stability of the prepared GNP-modified NPs (brine 0.9% w/v), stored at 4 °C was followed by DLS (Figure 13).

**Table 3. The analysis of GNP-modified picolinium NPs by DLS.**

| **Conc. of the block co polymer (mg/mL)** | **Hydrodynamic size (nm)** | **PDI** | **Concentration of GNPs (mg/mL)** | **Zeta potential (mV)** |
|---|---|---|---|---|
| 1.0 | 134 | 0.12 | 0.125 | - 3.44 |

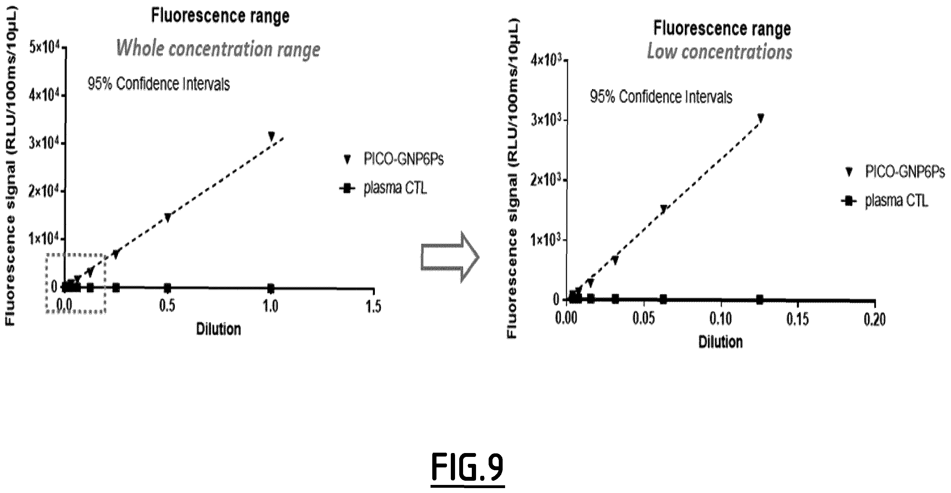

Further results in respect of GNP6Ps are illustrated in Figures 9-11.

Figures 9 shows that in the SWIR domain (ex: 808 nm, em: LP 1064 nm), PICO-GNP6Ps fluorescence detection was linear from dilution 1 to 1/256. At the highest dilution prepared (1/256), the detection limit was not reached yet (signal/background ratio = 2.65). Further, the solution is well detectable by SWIR fluorescence imaging and will allow efficient detection *in vivo* in mice. PICO-GNP6Ps were well detectable by SWIR fluorescence imaging with enough sensitivity to consider detection *in vivo* in mice.

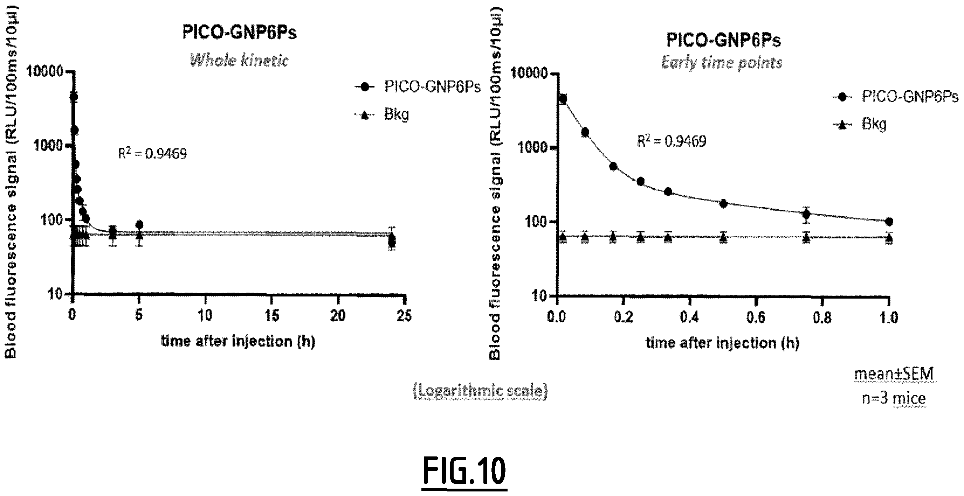

**Pharmacokinetics** of PICO-GNP6Ps was carried out in vivo in mice (Figure 10) : It was found that following intravenous injection, PICO-GNP6Ps display a short circulation time in the bloodstream : As soon as 1h post injection there is almost no more signal circulating in the bloodstream, with a half-life (Diffusion + elimination) of 2,31 min and a pure elimination half-life of 18,48 min.

**The intravenous injection** of 200 µL of PICO-GN6Ps in mice led to:
- No visible signs of discomfort during injection in healthy mice,
- No visible signs of pain or suffering during the awakening phase of the mice,
- Normal behavior of the mice up to 48h post injection.

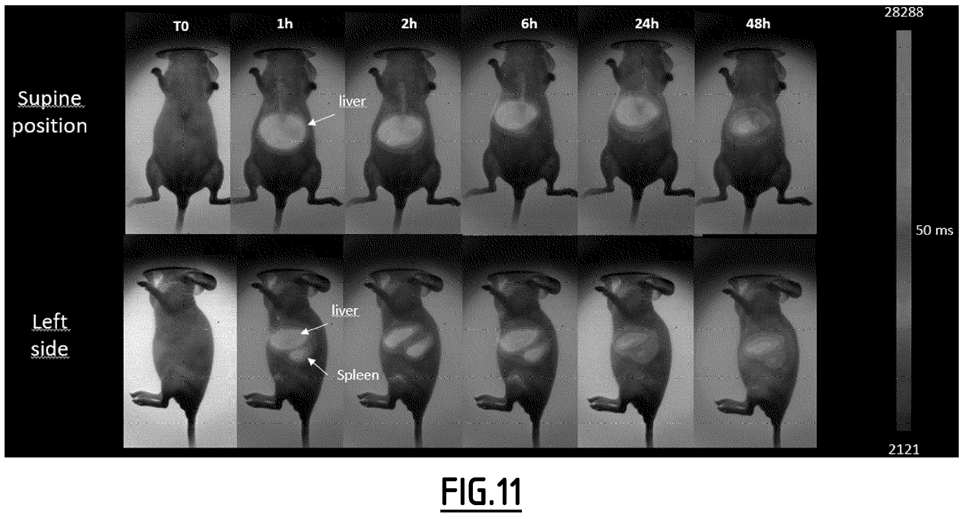

As illustrated in Figure 11, SWIR fluorescence imaging over 48 hours shows a strong liver signal as early as 1 hour. This signal decreases along time but remains significant at 48h post injection. Some fluorescence signal is also observed in the spleen but no further uptake is identified *in vivo* by noninvasive fluorescence imaging.

## Claims

1. A photo- or redox-cleavable amphiphilic copolymer compound of formula (I): 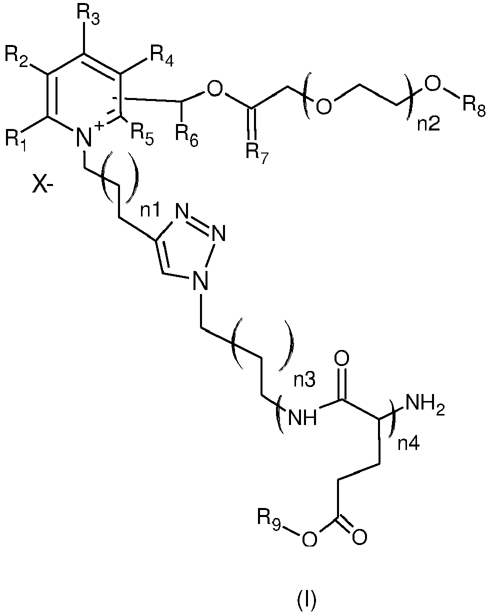 Where:
R₁, R₂, R₃ R₄ and R₅ substitution groups on the pyridinium ring are chosen from H, OH, OMethyl, CN, NR₂, NO₂, halogen, or two adjacent R₁, R₂, R₃ R₄ and R₅ are linked together to form one or more aromatic rings fused with the pyridinium ring to which they are attached so as to form an optionally substituted bi-cyclic quinolinium or optionally substituted fused tri-cyclic N-containing heteroaryl (e.g. acridinium),
R₆ is H or an alkyl group;
R₇ is O, or alkylene;
R₈ and R₉ identical or different are independently chosen from H, alkyl, aryl or benzyl, preferably methyl or benzyl;
Where the optional substituents are chosen from OH, OMethyl, CN, NO₂, NR₂, halogen;
R represents H, phenyl or a methyl group;
n₁ and n₃ are identical or different integers comprised between 0 and 5 defining the length of the spacer;
n₂ is an integer comprised between 34 and 80 denoting the polymerization degree of ethylene oxide in the hydrophilic block of the amphiphilic copolymer;
n₄ is an integer chosen between 18 and 40 denoting the polymerization degree of γ-benzyl-*L*-glutamate in the hydrophobic block of the amphiphilic copolymer;
X⁻ is a halide or a trifluoromethanesulfonate (OTf), phosphate, sulfate, perchlorate or nitrate counterion.

2. The photo- or redox-cleavable amphiphilic copolymer compound of formula (I) according to claim 1 chosen from: 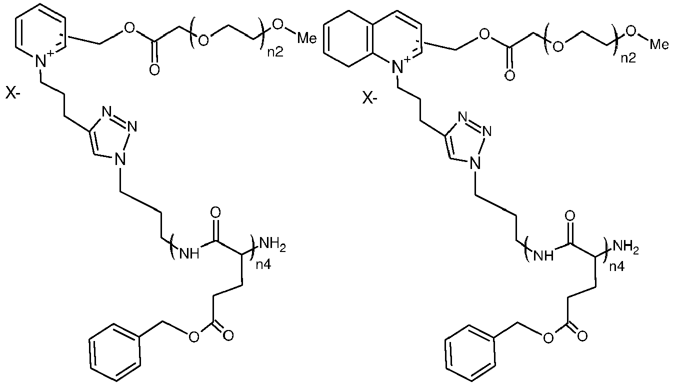 Where X⁻ is a halide or a trifluoromethanesulfonate (OTf⁻), and n2 and n4 are defined as in claim 1.

3. An amphiphilic copolymer vesicle, also called polymersome comprising an amphiphilic bilayer, wherein the amphiphilic bilayer of said vesicle comprises a photo-cleavable or redox-cleavable amphiphilic copolymer compound according to claim 1 or 2.

4. The polymersome according to claim 3, wherein it further comprises a mineral, i.e. a metal or a semi-metal, or an oxide or other chalcogenide nanoparticle embedded into said polymersome membrane.

5. The polymersome according to claim 4 wherein said mineral, metal or semi-metal, oxide or chalcogenide is chosen with constituent from the periodic table of the elements with an atomic number greater than 21, such as a transition metal (d group) , a noble metal, a metal alloy, a semi-metal, an alkali earth or a rare earth such as a lanthanide (f group).

6. The polymersome according to claim 4 or 5 wherein said mineral, metal or semi-metal, oxide or chalcogenide nanoparticle is an ultra-small iron oxide nanoparticle (USPIO), a superparamagnetic iron oxide nanoparticle (SPION), a very small iron oxide nanoparticles (VSION), a hafnium-oxide, an iron-bismuth or iron-platinum alloy, a gadolinium oxide, a dysprosium oxide, a bismuth selenide or bismuth telluride, silver, platinum, or a gold nanoparticle or atom cluster.

7. The polymersome according to anyone of claims 3 to 6 further comprising an active pharmaceutical ingredient (API), said API being encapsulated within either aqueous internal compartment or within hydrophobic membrane of said polymersome.

8. The polymersome according to claim 7, wherein said API is suitable for the treatment of cancers, inflammation, diabetes, bacterial and/or viral infections, orphan diseases.

9. An aqueous suspension comprising polymersomes according to anyone of claims 3 to 8.

10. A pharmaceutical composition or a medical device comprising an aqueous suspension according to claim 9.

11. A photo-cleavable or redox-cleavable amphiphilic copolymer compound, a polymersome, a suspension of polymersomes, a medical device or a pharmaceutical composition according to anyone of the preceding claims for use as a contrast agent for a bio-imaging modality, optical endoscopy, ultrasound echography, magnetic resonance imaging (MRI), X-ray scanner also called computed tomography (CT) combined with a drug carrier for the image-guided treatment of a disorder, wherein said treatment comprises:
- the implantation of said medical device at a disorder site or the systemic administration of said compound, polymersome, suspension of polymersomes or pharmaceutical composition;
- the activation of the compound of formula (I) at the targeted site by either an external mean (irradiation, electromagnetic field... ) or by an endogenous signal thereby releasing the API at the targeted site; and
- optionally monitoring the distribution of the mineral, metal or semi-metal, oxide or chalcogenide embedded in polymersomes within the body by a noninvasive bio-imaging technique.

12. The compound, polymersome, suspension, medical device or pharmaceutical composition, for use according to claim 11 wherein the activation is achieved by cleavage of the polymersome membrane induced by UV light, visible photon (photodynamic), ionizing (beta ray, X-ray or gamma) irradiation, ultrasound or endogenous redox activation at the targeted site in the body.

13. The compound, polymersome, suspension, medical device or pharmaceutical composition, for use according to claim 11 or 12 wherein:
- said disorder is a cancer, immune-related diseases, inflammation, diabetes, bacterial and/or viral infections, pediatric or age-related diseases,
- the polymersome encapsulates an active pharmaceutical ingredient (API),
- the API is chosen from APIs suitable for the treatment of cancer, immune-related diseases, inflammation, diabetes, bacterial and/or viral infections, pediatric or age-related diseases, such as Sorafenib^{®} Doxorubicin, Monomethyl auristatin E (MMAE), cisplatin, or Combretastatin^{®}, and
- the activation is carried out by beta rays, X rays or gamma irradiation, or by exploiting exogenous or endogenous redox signals such as redox enzymes, metalloproteases, reactive oxygen species (ROS) like peroxides or reactive nitrogen species (NO.).

14. Process of preparation of the copolymer according to anyone of claims 1 to 2 comprising the step of reacting a compound of formula (II): 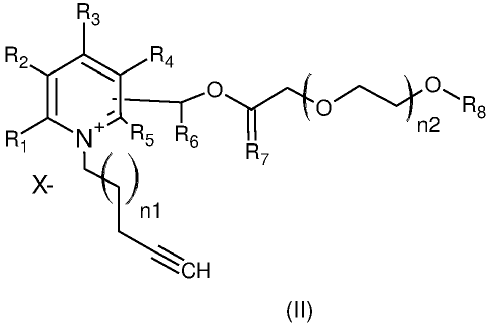 with a compound of formula (III)°: 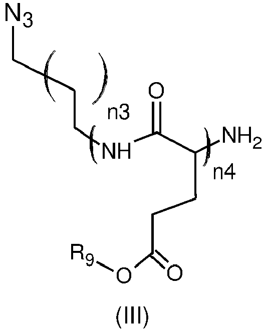 Wherein R₁, R₂, R₃ R₄, R₅, R₆, R₇, R₈, R₉, R, X, n₁, n₂ n₃ and n₄ are defined as in anyone of claims 1 to 2.

15. Process of preparation of the suspension of polymersomes according to anyone of claims 3 to 8 comprising:
- Optionally mixing a compound of formula (I) with a hydrophobically coated mineral, metal or semi-metal, oxide or chalcogenide nanoparticle suspension and/or an API in an organic solvent miscible with water,
- Self-assembly by nanoprecipitation of the polymersome vesicles in an aqueous solution (also called "solvent-shift" method), optionally followed by
- dialysis of the suspension or heating to remove any trace of organic solvent.

## Patentansprüche

1. Photo- oder redoxspaltbare amphiphile Copolymerverbindung von Formel (I): 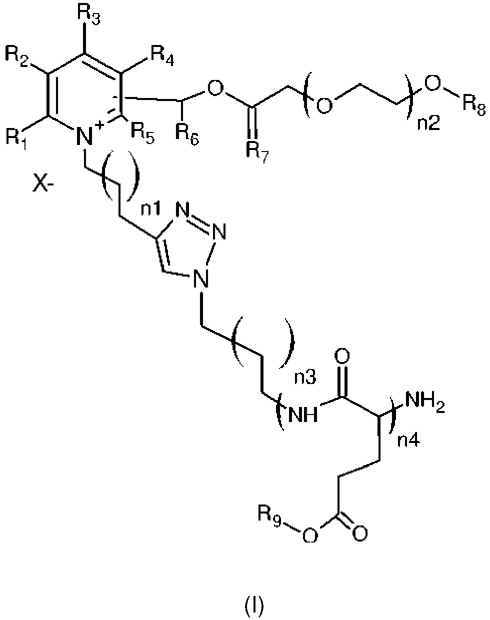 wobei:
die Substitutionsgruppen R₁, R₂, R₃ R₄ und R₅ an dem Pyridiniumring ausgewählt sind aus H, OH, OMethyl, CN, NR₂, NO₂, Halogen, oder zwei benachbarte R₁, R₂, R₃ R₄ und R₅ miteinander verbunden sind, um einen oder mehrere aromatische Ringe zu bilden, die mit dem Pyridiniumring, an den sie angelagert sind, kondensiert sind, um ein optional substituiertes bi-cyclisches Chinolinium oder ein optional substituiertes kondensiertes tri-cyclisches N-haltiges Heteroaryl (z. B. Acridinium) zu bilden,
R₆ H oder eine Alkylgruppe ist;
R₇ O oder Alkylen ist;
R₈ und R₉, die gleich oder verschieden sind, unabhängig ausgewählt sind aus H, Alkyl, Aryl oder Benzyl, vorzugsweise Methyl oder Benzyl;
wobei die fakultativen Substituenten ausgewählt sind aus OH, OMethyl, CN, NO₂, NR₂ und Halogen;
R H, Phenyl oder eine Methylgruppe darstellt;
n₁ und n₃ gleiche oder verschiedene ganze Zahlen zwischen 0 und 5 sind, die die Länge des Abstandhalters definieren;
n₂ eine ganze Zahl zwischen 34 und 80 ist, die den Polymerisationsgrad von Ethylenoxid in dem hydrophilen Block des amphiphilen Copolymers angibt;
n₄ eine ganze Zahl zwischen 18 und 40 ist, die den Polymerisationsgrad von y-Benzyl-L-glutamat in dem hydrophoben Block des amphiphilen Copolymers angibt;
X⁻ ein Halogenid oder ein Trifluormethansulfonat (OTf), Phosphat, Sulfat, Perchlorat oder Nitrat-Gegenion ist.

2. Photo- oder redoxspaltbare amphiphile Copolymerverbindung von Formel (I) nach Anspruch 1, ausgewählt aus: 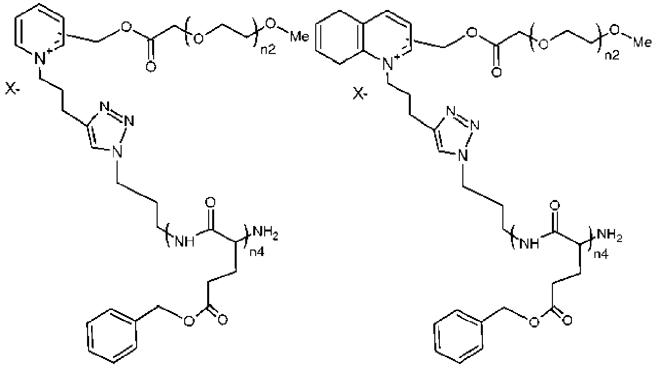 wobei X⁻ ein Halogenid oder ein Trifluormethansulfonat (OTf⁻) ist und n2 und n4 wie definiert in Anspruch 1 sind.

3. Amphiphiles Copolymervesikel, auch Polymersom genannt, umfassend eine amphiphile Doppelschicht, wobei die amphiphile Doppelschicht des Vesikels eine photospaltbare oder redoxspaltbare amphiphile Copolymerverbindung nach Anspruch 1 oder 2 umfasst.

4. Polymersom nach Anspruch 3, **dadurch gekennzeichnet, dass** es ferner ein Mineral, d. h. ein Metall oder ein Halbmetall, oder ein Oxid oder ein anderes Chalkogenid-Nanopartikel umfasst, das in die Polymersom-Membran eingebettet ist.

5. Polymersom nach Anspruch 4, wobei das Mineral, das Metall oder Halbmetall, das Oxid oder das Chalkogenid mit einem Bestandteil aus dem Periodensystem der Elemente mit einer Ordnungszahl von mehr als 21 ausgewählt ist, wie beispielsweise ein Übergangsmetall (Gruppe d), ein Edelmetall, eine Metalllegierung, ein Halbmetall, eine Erdalkalie oder eine seltene Erde, wie beispielsweise ein Lanthanid (Gruppe f).

6. Polymersom nach Anspruch 4 oder 5, wobei das Mineral-, Metall- oder Halbmetall-, Oxid- oder Chalkogenid-Nanopartikel ein ultrakleines Eisenoxid-Nanopartikel (USPIO), ein superparamagnetisches Eisenoxid-Nanopartikel (SPION), ein sehr kleines Eisenoxid-Nanopartikel (VSION), ein Hafniumoxid, eine Eisen-Wismut- oder Eisen-Platin-Legierung, ein Gadoliniumoxid, ein Dysprosiumoxid, ein Wismutselenid oder Wismuttellurid, Silber, Platin oder ein Gold-Nanopartikel oder -Atomcluster ist.

7. Polymersom nach einem der Ansprüche 3 bis 6, ferner umfassend einen pharmazeutischen Wirkstoff (API), wobei der API entweder in einem wässrigen inneren Kompartiment oder in einer hydrophoben Membran des Polymersoms eingekapselt ist.

8. Polymersom nach Anspruch 7, wobei der API für die Behandlung von Krebserkrankungen, Entzündungen, Diabetes, bakteriellen und/oder viralen Infektionen und seltenen Krankheiten geeignet ist.

9. Bereitstellen einer Suspension umfassend Polymersome nach einem der Ansprüche 3 bis 8.

10. Pharmazeutische Zusammensetzung oder medizinische Vorrichtung, umfassend eine wässrige Suspension nach Anspruch 9.

11. Photospaltbare oder redoxspaltbare amphiphile Copolymerverbindung, ein Polymersom, eine Suspension von Polymersomen, eine medizinische Vorrichtung oder eine pharmazeutische Zusammensetzung nach einem der vorherigen Ansprüche zur Verwendung als Kontrastmittel für eine Bio-Bildgebungsmodalität, optische Endoskopie, Ultraschallechographie, Magnetresonanztomographie (MRI), Röntgenscanner, auch Computertomographie (CT) genannt, in Kombination mit einem Arzneimittelträger für die bildgesteuerte Behandlung einer Erkrankung, wobei die Behandlung Folgendes umfasst:
- die Implantation der medizinischen Vorrichtung an der Stelle einer Erkrankung oder die systemische Verabreichung der Verbindung, des Polymersoms, der Suspension von Polymersomen oder der pharmazeutischen Zusammensetzung;
- die Aktivierung der Verbindung von Formel (I) an der Zielstelle entweder durch eine externe Einrichtung (Bestrahlung, elektromagnetisches Feld...) oder durch ein endogenes Signal, wodurch der API an der Zielstelle freigesetzt wird; und
- optional Überwachen der Verteilung des Minerals, Metalls oder Halbmetalls, Oxids oder Chalkogenids, das in Polymersomen eingebettet ist, in dem Körper durch ein nicht invasives bio-bildgebendes Verfahren.

12. Verbindung, Polymersom, Suspension, medizinische Vorrichtung oder pharmazeutische Zusammensetzung zur Verwendung nach Anspruch 11, wobei die Aktivierung durch Spaltung der Polymersommembran erreicht wird, die durch UV-Licht, sichtbare Photonen (photodynamisch), ionisierende Strahlung (Betastrahlung, Röntgenstrahlung oder Gammastrahlung), Ultraschall oder endogene Redoxaktivierung an der Zielstelle in dem Körper induziert wird.

13. Verbindung, Polymersom, Suspension, medizinische Vorrichtung oder pharmazeutische Zusammensetzung zur Verwendung nach Anspruch 11 oder 12, wobei:
- die Erkrankung Krebs, immunbedingte Krankheiten, Entzündung, Diabetes, bakterielle und/oder virale Infektionen, pädiatrische oder altersbedingte Krankheiten sind,
- das Polymersom einen pharmazeutischen Wirkstoff (API) verkapselt,
- der API ausgewählt ist aus APIs, die für die Behandlung von Krebs, immunbedingten Krankheiten, Entzündungen, Diabetes, bakteriellen und/oder viralen Infektionen, pädiatrischen oder altersbedingten Krankheiten geeignet sind, wie beispielsweise Sorafenib^{®} Doxorubicin, Monomethylauristatin E (MMAE), Cisplatin oder Combretastatin^{®}, und
- die Aktivierung durch Beta-, Röntgen- oder Gammastrahlen oder durch die Nutzung exogener oder endogener Redox-Signale, wie beispielsweise Redox-Enzyme, Metalloproteasen, reaktive Sauerstoffspezies (ROS), wie beispielsweise Peroxide oder reaktive Stickstoffspezies (NO•) durchgeführt wird.

14. Verfahren zur Herstellung des Copolymers nach einem der Ansprüche 1 bis 2, umfassend den Schritt eines Reagierens einer Verbindung von Formel (II): 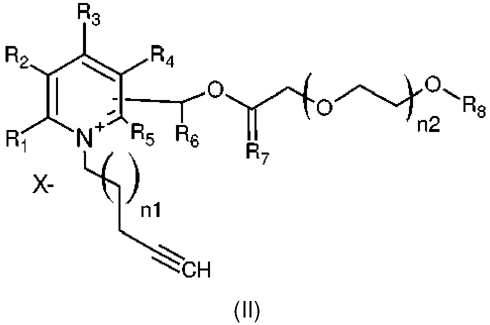 mit einer Verbindung von Formel (III)°: 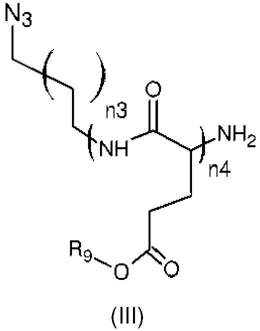 wobei R₁, R₂, R₃, R₄, R₅, R₆, R₇, R₈, R₉, R, X, n₁, n₂ n₃ und n₄ wie definiert in einem der Ansprüche 1 bis 2 sind.

15. Herstellungsverfahren einer Suspension von Polymersomen nach einem der Ansprüche 3 bis 8, umfassend:
- Optionales Mischen einer Verbindung von Formel (I) mit einer hydrophob beschichteten Suspension von Mineral-, Metall- oder Halbmetall-, Oxid- oder Chalkogenid-Nanopartikeln und/oder einem API in einem Lösungsmittel, das mit Wasser mischbar ist,
- Selbstorganisation durch Nanopräzipitation der Polymersomen-Vesikel in einer wässrigen Lösung (auch bezeichnet als "Solvent-Shift"-Verfahren), optional gefolgt von
- Dialyse der Suspension oder Erhitzen, um alle Spuren von organischem Lösungsmittel zu entfernen.

## Revendications

1. Composé copolymère amphiphile photo-clivable ou clivable par oxydoréduction de 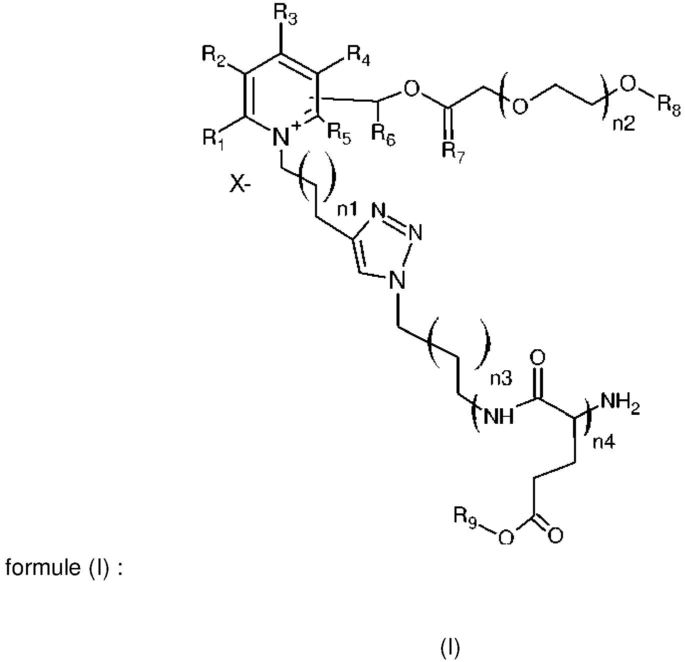 où :
les groupes de substitution R₁, R₂, R₃, R₄ et R₅ sur le cycle de pyridinium sont choisis parmi H, OH, OMéthyle, CN, NR₂, NO₂, un halogène, ou deux groupes R₁, R₂, R₃, R₄ et R₅ adjacents sont liés ensemble pour former un ou plusieurs cycles aromatiques fusionnés avec le cycle de pyridinium auquel ils sont attachés de manière à former un quinolinium bicyclique facultativement substitué ou un hétéroaryle tricyclique fusionné contenant un ou plusieurs atomes de N facultativement substitué (par exemple l'acridinium),
R₆ est H ou un groupe alkyle ;
R₇ est O ou un alkylène ;
R₈ et R₉ identiques ou différents sont choisis indépendamment parmi H, un alkyle, un aryle ou un benzyle, de préférence un méthyle ou un benzyle ;
où les substituants facultatifs sont choisis parmi OH, OMéthyl, CN, NO₂, NR₂, un halogène ;
R représente H, un phényle ou un groupe méthyle ;
n₁ et n₃ sont des nombres entiers identiques ou différents compris entre 0 et 5 définissant la longueur de l'espaceur ;
n₂ est un nombre entier compris entre 34 et 80 représentant le degré de polymérisation de l'oxyde d'éthylène dans le bloc hydrophile du copolymère amphiphile ;
n₄ est un nombre entier choisi entre 18 et 40 représentant le degré de polymérisation du y-benzyl-*L*-glutamate dans le bloc hydrophobe du copolymère amphiphile ;
X⁻ est un contre-ion halogénure ou trifluorométhanesulfonate (OTf⁻), phosphate, sulfate, perchlorate ou nitrate.

2. Composé copolymère amphiphile photo-clivable ou clivable par oxydoréduction de formule (I) selon la revendication 1 choisi parmi : 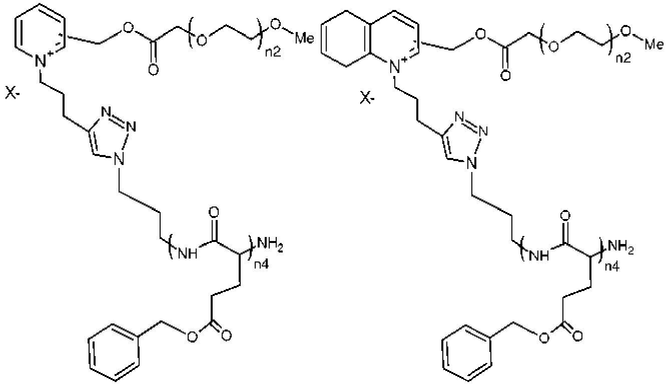 où X est un halogénure ou un trifluorométhanesulfonate (OTf⁻), et n2 et n4 sont définis comme dans la revendication 1.

3. Vésicule de copolymère amphiphile, également appelée polymersome, comprenant une bicouche amphiphile, dans laquelle la bicouche amphiphile de ladite vésicule comprend un composé de copolymère amphiphile photo-clivable ou clivable par oxydoréduction selon la revendication 1 ou 2.

4. Polymersome selon la revendication 3, dans lequel il comprend en outre une nanoparticule minérale, c'est-à-dire métallique ou semi-métallique, ou d'oxyde ou d'un autre chalcogénure incorporée dans ladite membrane de polymersome.

5. Polymersome selon la revendication 4 dans lequel ledit minéral, métal ou semi-métal, oxyde ou chalcogénure est choisi parmi les constituants du tableau périodique des éléments de numéro atomique supérieur à 21, tels qu'un métal de transition (groupe d), un métal noble, un alliage métallique, un semi-métal, une terre alcaline ou une terre rare telle qu'un lanthanide (groupe f).

6. Polymersome selon la revendication 4 ou 5 dans lequel ladite nanoparticule minérale, métallique ou semi-métallique, d'oxyde ou de chalcogénure est une nanoparticule d'oxyde de fer ultra-petite (USPIO), une nanoparticule d'oxyde de fer superparamagnétique (SPION), une nanoparticule d'oxyde de fer de très petite taille (VSION), un oxyde d'hafnium, un alliage fer-bismuth ou fer-platine, un oxyde de gadolinium, un oxyde de dysprosium, un séléniure de bismuth ou un tellurure de bismuth, de l'argent, du platine ou une nanoparticule ou un amas d'atomes d'or.

7. Polymersome selon l'une des revendications 3 à 6 comprenant en outre un principe actif pharmaceutique (PAP), ledit PAP étant encapsulé dans le compartiment interne aqueux ou dans la membrane hydrophobe dudit polymersome.

8. Polymersome selon la revendication 7, dans lequel ledit PAP convient au traitement des cancers, de l'inflammation, du diabète, des infections bactériennes et/ou virales, des maladies orphelines.

9. Suspension aqueuse comprenant des polymersomes selon l'une quelconque des revendications 3 à 8.

10. Composition pharmaceutique ou dispositif médical comprenant une suspension aqueuse selon la revendication 9.

11. Composé copolymère amphiphile photo-clivable ou clivable par oxydoréduction, polymersome, suspension de polymersomes, dispositif médical ou composition pharmaceutique selon l'une quelconque des revendications précédentes pour utilisation comme agent de contraste pour une modalité de bio-imagerie, endoscopie optique, échographie, imagerie par résonance magnétique (IRM), scanner à rayons X également appelé tomodensitométrie (TDM) combiné à un vecteur de médicament pour le traitement guidé par l'image d'un trouble, dans lequel ledit traitement comprend :
- l'implantation dudit dispositif médical sur un site de trouble ou l'administration systémique dudit composé, dudit polymersome, de ladite suspension de polymersomes ou de ladite composition pharmaceutique ;
- l'activation du composé de formule (I) au niveau du site ciblé par un moyen externe (irradiation, champ électromagnétique...) ou par un signal endogène, libérant ainsi le PAP au niveau du site ciblé ; et
- facultativement, la surveillance de la distribution du minéral, du métal ou semi-métal, de l'oxyde ou du chalcogénure incorporé dans des polymersomes à l'intérieur du corps par une technique de bio-imagerie non invasive.

12. Composé, polymersome, suspension, dispositif médical ou composition pharmaceutique, pour utilisation selon la revendication 11 dans lequel l'activation est obtenue par clivage de la membrane de polymersome induit par la lumière UV, les photons visibles (photodynamique), l'irradiation ionisante (rayons bêta, rayons X ou gamma), les ultrasons ou l'activation redox endogène au niveau du site ciblé dans l'organisme.

13. Composé, polymersome, suspension, dispositif médical ou composition pharmaceutique, pour utilisation selon la revendication 11 ou 12, dans lequel :
- ledit trouble est un cancer, des maladies liées au système immunitaire, une inflammation, un diabète, des infections bactériennes et/ou virales, des maladies pédiatriques ou des maladies liées à l'âge,
- le polymersome encapsule un principe actif pharmaceutique (PAP),
- le PAP est choisi parmi les PAPs adaptés au traitement du cancer, des maladies liées au système immunitaire, de l'inflammation, du diabète, des infections bactériennes et/ou virales, des maladies pédiatriques ou liées à l'âge, tels que Sorafenib^{®} Doxorubicin, la monométhylauristatine E (MMAE), le cisplatine, ou Combretastatin^{®}, et
- l'activation est réalisée par des rayons bêta, des rayons X ou une irradiation gamma, ou en exploitant des signaux redox exogènes ou endogènes tels que des enzymes redox, des métalloprotéases, des espèces réactives de l'oxygène (ROS) comme les peroxydes ou les espèces réactives de l'azote (NO•).

14. Procédé de préparation du copolymère selon l'une quelconque des revendications 1 et 2 comprenant l'étape de mise en réaction d'un composé de formule (II) : 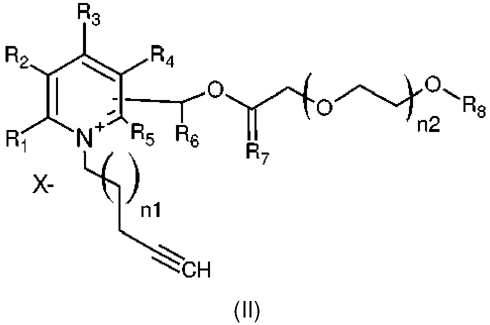 avec un composé de la formule (III)° : 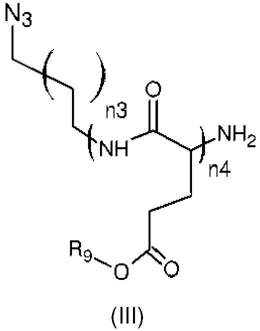 où R₁, R₂, R₃, R₄, R₅, R₆, R₇, R₈, R₉, R, X, n₁, n₂,n₃ et n₄ sont définis comme dans l'une quelconque des revendications 1 et 2.

15. Procédé de préparation de la suspension de polymersomes selon l'une quelconque des revendications 3 à 8 comprenant :
- le mélange facultatif d'un composé de formule (I) avec une suspension de nanoparticules minérales, métalliques ou semi-métalliques, d'oxyde ou de chalcogénure à revêtement hydrophobe et/ou un PAP dans un solvant organique miscible avec l'eau,
- l'auto-assemblage par nanoprécipitation des vésicules de polymersomes dans une solution aqueuse (également appelée procédé « déplacement de solvant »), facultativement suivi d'une
- dialyse de la suspension ou chauffage pour éliminer toute trace de solvant organique.
